(19) Europäisches Patentamt
European Patent Office
Office européen des brevets

(11) EP 1 418 167 B1

(12) # EUROPEAN PATENT SPECIFICATION

(45) Date of publication and mention of the grant of the patent:
**26.01.2005 Bulletin 2005/04**

(51) Int Cl.[7]: **C07C 59/42**, C07C 59/46, A61K 31/202, A61P 27/02

(21) Application number: **04003004.1**

(22) Date of filing: **23.10.2000**

(54) **Hydroxyeicosatetraenoic acid analogs and methods of their use in treating dry eye disorders**

Hydroxyeicosatetraensäureanaloge und Verfahren deren Verwendung zur Behandlung des trockenen Auges

Analogues de l'acide hydroxyeicosatetraenoique et procédés d'utilisation de ces derniers dans le traitement de l'oeil sec

(84) Designated Contracting States:
**AT BE CH CY DE DK ES FI FR GB GR IE IT LI LU MC NL PT SE**

(30) Priority: **09.11.1999 US 164386 P**
**09.11.1999 US 164369 P**
**09.11.1999 US 164371 P**

(43) Date of publication of application:
**12.05.2004 Bulletin 2004/20**

(62) Document number(s) of the earlier application(s) in accordance with Art. 76 EPC:
**00973791.7 / 1 228 029**

(73) Proprietor: **Alcon Inc.**
**6331 Hünenberg (CH)**

(72) Inventors:
- **Falck, John, R.**
  **Dallas, Texas 75225 (US)**
- **Conrow, Raymond, E.**
  **Crowley, Texas 76036 (US)**
- **Hellberg, Mark, R.**
  **Arlington, Texas 76017 (US)**
- **Klimko, Peter, G.**
  **Fort Worth, Texas 76110 (US)**

(74) Representative: **Best, Michael, Dr. et al**
**Lederer & Keller**
**Patentanwälte**
**Prinzregentenstrasse 16**
**80538 München (DE)**

(56) References cited:

**US-A- 4 906 467** **US-A- 5 455 265**
**US-A- 5 696 166**

- **STEFFENRUD S ET AL: "GAS CHROMATOGRAPHY-MASS SPECTROMETRY OF MONOHYDROXYEICOSATETRAENOIC ACIDS AS THEIR METHYL ESTERS TRIMETHYLSILYL, ALLYLDIMETHYLSILYL AND TERT.-BUTYLDIMETHYLSILYL ETHERS" JOURNAL OF CHROMATOGRAPHY, ELSEVIER SCIENCE PUBLISHERS B.V. AMSTERDAM, NL, vol. 416, no. 2, 1987, pages 219-235, XP000990117 ISSN: 0021-9673**
- **LOW C-E ET AL: "INHIBITION OF PHYTOHEMAGGLUTININ-INDUCED LYMPHOCYTE MITOGENESIS BY LIPOXYGENASE METABOLITES OF ARACHIDONIC ACID: STRUCTURE-ACTIVITY RELATIONSHIPS" JOURNAL OF LIPID RESEARCH, BETHESDA, MD, US, vol. 25, no. 10, October 1984 (1984-10), pages 1090-1095, XP000990320 ISSN: 0022-2275**
- **PETRICH K ET AL: "THE SUPPRESSION OF 5-LIPOXYGENATION OF ARACHIDONIC ACID IN HUMAN POLYMORPHONUCLEAR LEUCOCYTES BY THE 15-LIPOXYGENASE PRODUCT (15S)-HYDROXY-(5Z,8Z,11Z,13E)-EICOSATETR AE NOIC ACID: STRUCTURE-ACTIVITY RELATIONSHIP AND MECHANISM OF ACTION" BIOCHEMICAL JOURNAL, PORTLAND PRESS, LONDON, GB, vol. 314, no. 3, 15 March 1996 (1996-03-15), pages 911-916, XP000990343 ISSN: 0264-6021**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 418 167 B1

## Description

The present invention is directed to compositions containing hydroxyeicosatetraenoic acid analogs and to the preparation of a medicament for the treatment of dry eye.

### Background of the Invention

Dry eye, also known generically as *keratoconjunctivitis sicca*, is a common ophthalmological disorder affecting millions of Americans each year (Schein *et. al*., *Prevalence of dry eye among the elderly.* American J. Ophthalmology, 124:723-738, (1997)). The condition is particularly widespread among post-menopausal women due to hormonal changes following the cessation of fertility. Dry eye may afflict an individual with varying severity. In mild cases, a patient may experience burning, a feeling of dryness, and persistent irritation such as is often caused by small bodies lodging between the eyelid and the eye surface. In severe cases, vision may be substantially impaired. Other diseases, such as Sjogren's disease and *cicatricial pemphigoid* manifest dry eye complications.

Although it appears that dry eye may result from a number of unrelated pathogenic causes, all presentations of the complication share a common effect, that is the breakdown of the pre-ocular tear film, which results in dehydration of the exposed outer surface and many of the symptoms outlined above (Lemp, *Report of the Nation Eye Institute/ Industry Workshop on Clinical Trials in Dry Eyes,* The CLAO Journal, volume 21, number 4, pages 221-231 (1995)). Four events have been identified which singly or in combination are believed to result in the dry eye condition: a) decreased tear production or increased tear evaporation; b) decreased conjunctival goblet-cell density; c) increased corneal desquamation; and d) destabilization of the cornea-tear interface (Gilbard, *Dry eye: pharmacological approaches*, *effects*, *and progress*. The CLAO Journal, 22:141-145 (1996)). Another major problem is the decreased mucin production by the conjunctival cells and/or corneal epithelial cells of mucin, which protects and lubricates the ocular surface (Gipson and Inatomi, *Mucin genes expressed by ocular surface epithelium*. Progress in Retinal and Eye Research, 16:81-98 (1997)).

Practitioners have taken several approaches to the treatment of dry eye. One common approach has been to supplement and stabilize the ocular tear film using so-called artificial tears instilled throughout the day. Another approach has been the use of ocular inserts that provide a tear substitute or to stimulate endogenous tear production.

Examples of the tear substitution approach include the use of buffered, isotonic saline solutions, aqueous solutions containing water-soluble polymers that render the solutions more viscous and thus less easily shed by the eye. Tear reconstitution is also attempted by providing one or more components of the tear film such as phospholipids and oils. Examples of these treatment approaches are disclosed in United States Patent Nos. 4,131,651 (Shah et al.), 4,370,325 (Packman), 4,409,205 (Shively), 4,744,980 and 4,883,658 (Holly), 4,914,088 (Glonek), 5,075,104 (Gressel et al.) and 5,294,607 (Glonek et al.).

United States Patents directed to the use of ocular inserts in the treatment of dry eye include 3,991,759 (Urquhart). Other semi-solid therapy has included the administration of carrageenans (5,403,841, Lang) which gel upon contact with naturally occurring tear film.

Another recent approach involves the provision of lubricating substances in lieu of artificial tears. United States Patent No. 4,818,537 (Guo) discloses the use of a lubricating, liposome-based composition.

Aside from the above efforts, which are directed primarily to the alleviation of symptoms associated with dry eye, methods and compositions directed to treatment of the dry eye condition have also been pursued. For example, United States Patent No. 5,041,434 (Lubkin) discloses the use of sex steroids, such as conjugated estrogens, to treat dry eye condition in post-menopausal women; United States Patent No. 5,290,572 (MacKeen) discloses the use of finely divided calcium ion compositions to stimulate preocular tear film; and United States Patent No. 4,966,773 (Gressel et al.) discloses the use of microfine particles of one or more retinoids for ocular tissue normalization.

Although these approaches have met with some success, problems in the treatment of dry eye nevertheless remain. The use of tear substitutes, while temporarily effective, generally requires repeated application over the course of a patient's waking hours. It is not uncommon for a patient to have to apply artificial tear solution ten to twenty times over the course of the day. Such an undertaking is not only cumbersome and time consuming, but is also potentially very expensive. Transient symptoms of dry eye associated with refractive surgery have been reported to last in some cases from six weeks to six months or more following surgery.

The use of ocular inserts is also problematic. Aside from cost, they are often unwieldy and uncomfortable. Further, as foreign bodies introduced in the eye, they can be a source of contamination leading to infections. In situations where the insert does not itself produce and deliver a tear film, artificial tears must still be delivered on a regular and frequent basis.

In view of the foregoing, there is a clear need for an effective treatment for dry eye that is capable of alleviating symptoms, as well as treating the underlying physical and physiological deficiencies of dry eye, and that is both convenient and inexpensive to administer.

Mucins are proteins that are heavily glycosylated with glucosamine-based moieties. Mucins provide protective and lubricating effects to epithelial cells, especially those of mucosal membranes. Mucins have been shown to be secreted by vesicles and discharged on the surface of the conjunctival epithelium of human eyes (Greiner et al., *Mucus Secretory Vesicles in Conjunctival Epithelial Cells of Wearers of Contact Lenses,* Archives of Ophthalmology, volume 98, pages 1843-1846 (1980); and Dilly et al., *Surface Changes in the Anaesthetic Conjunctiva in Man, with Special Reference to the Production of Mucus from a Non-Goblet-Cell Source,* British Journal of Ophthalmology, volume 65, pages 833-842 (1981)). A number of human-derived mucins which reside in the apical and subapical corneal epithelium have been discovered and cloned (Watanabe et al., *Human Corneal and conjunctival Epithelia Produce a Mucin-Like Glycoprotein for the Apical Surface*, Investigative Ophthalmology and Visual Science, volume 36, number 2, pages 337-344 (1995)). Recently, Watanabe discovered a new mucin which is secreted via the cornea apical and subapical cells as well as the conjunctival epithelium of the human eye (Watanabe et al., IOVS, volume 36, number 2, pages 337-344 (1995)). These mucins provide lubrication, and additionally attract and hold moisture and sebaceous material for lubrication and the corneal refraction of light.

Mucins are also produced and secreted in other parts of the body including lung airway passages, and more specifically from goblet cells interspersed among tracheal/bronchial epithelial cells. Certain arachidonic acid metabolites have been shown to stimulate mucin production in these cells. Yanni reported the increased secretion of mucosal glycoproteins in rat lung by hydroxyeicosatetraenoic acid ("HETE") derivatives (Yanni et al, *Effect of Intravenously Administered Lipoxygenase Metabolites on Rat Tracheal Mucous Gel Layer Thickness,* International Archives of Allergy And Applied Immunology, volume 90, pages 307-309 (1989)). Similarly, Marom has reported the production of mucosal glycoproteins in human lung by HETE derivatives (Marom et al., *Human Airway Monohydroxy- eicosatetraenoic Acid Generation and Mucus Release,* Journal of Clinical Investigation, volume 72, pages 122-127 (1983)). Nowhere in the art, however, has the use of HETE derivatives been proposed to stimulate mucin production in ocular tissues as a treatment for dry eye.

The conventional treatment for dry eye, as discussed above, includes administration of artificial tears to the eye several times a day. Other agents claimed for increasing ocular mucin and/or tear production include vasoactive intestinal polypeptide (Dartt *et. al.*, *Vasoactive intestinal peptide-stimulated glycoconjugate secretion from conjunctival goblet cells.* Experimental Eye Research, 63:27-34, (1996)) , gefarnate (Nakmura *et. al., Gefarnate stimulates secretion of mucin-like glycoproteins by corneal epithelium in vitro and protects corneal epithelium from desiccation in vivo*, Experimental Eye Research, 65:569-574 (1997)), and the use of liposomes (U.S. Patent No. 4,818,537), androgens (U.S. Patent No. 5,620,921), melanocyte stimulating hormones (U.S. Patent No. 4,868,154), and phosphodiesterase inhibitors (U.S. Patent No. 4,753,945), retinoids (U.S. Patent No. 5,455,265). However, many of these compounds or treatments suffer from a lack of specificity, efficacy and potency and none of these agents have been marketed so far as therapeutically useful products to treat dry eye and related ocular surface diseases. Of particular relevance to the present invention is the claimed use of hydroxyeicosatetraenoic acid derivatives to treat dry eye (U.S. Patent No. 5,696,166). Thus, there remains a need for an efficacious therapy for the treatment of dry eye and related diseases.

## Summary of the Invention

The present invention is directed to compositions and to the preparation of a medicament for the treatment of dry eye and other disorders requiring the wetting of the eye, including symptoms of dry eye associated with refractive surgery such as LASIK surgery. More specifically, the present invention discloses analogs of (5*Z*,8*Z*,11*Z*,13*E*)-15-hydroxyeicosa-5,8,11,14-tetraenoic acid (15-HETE) and their use in the preparation of a medicament for treating dry eye type disorders. The compositions are preferably administered topically to the eye.

## Detailed Description of the Invention

It has now been discovered that certain 15-HETE analogs are useful in treating dry eye or other disorders requiring the wetting of the eye. It is believed that such analogs stimulate mucin production in human conjunctival epithelium. These compounds are of formula **I**:

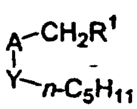

**I**

wherein:

$R^1$ is $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$, or 2,3,4,5-tetrazol-1-yl, where:

R is H or a pharmaceutically acceptable cation, or $CO_2R$ forms a pharmaceutically acceptable ester moiety;
$NR^2R^3$, $NR^5R^6$ are the same or different and comprise a free or functionally modified amino group;
$OR^4$ comprises a free or functionally modified hydroxy group;
Hal is F, Cl, Br, or I;
$R^{20}$ is H, alkyl, acyl;
$R^{21}$ is H or a pharmaceutically acceptable cation, or $COSR^{21}$ forms a pharmaceutically acceptable thioester moiety;

A is $L_1$-$A_2$-$L_2$, $L_3$-$A_2$-$L_4$, or $L_5$-$A_2$-$L_3$;
$A_2$ is

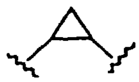 ;

$L_1$ is $CH_2$-B-D;
B and D are the same or different and are $CH_2CH_2$, CH=CH, or C≡C;
$L_2$ is $CH_2$-K-$CH_2CH_2$;
K is $CH_2CH_2$, CH=CH, or C≡C;
$L_3$ is $CH_2CH_2CH_2$, $CH_2CH$=CH, $CH_2C$≡C, CH=$CHCH_2$, C≡$CCH_2$, or CH=C=CH;
$L_4$ is X-$CH_2CH_2$;
X is $CH_2CH_2CH$=CH, $CH_2CH_2C$≡C, $CH_2CH_2CH_2CH_2$, $CH_2CH$=$CHCH_2$, $CH_2C$≡$CCH_2$, CH=$CHCH_2CH_2$, C≡$CCH_2CH_2$, $CH_2CH$=C=CH, or CH=C=$CHCH_2$;
$L_5$ is $CH_2CH_2$-B-D; and
Y is C(O) (*i.e.* a carbonyl group) or Y is

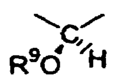
or
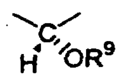 ;

wherein $R^9O$ constitutes a free or functionally modified hydroxy group.

Compound **1** (see example 1 below) has been identified as an arachidonic acid metabolite in human blood [Evans and Sprecher, Prostaglandins, 29:431 (1985)].

The compounds of formula **I** may also be incorporated into phospholipids as glyceryl esters or sphingomyelin amides. Phospholipid sphingomyelin amides of the compounds of formula **I** will typically comprise a formula **I** compound amidated *via* its carbon 1 carboxylate to the amino group of the sphingomyelin backbone. The phospholipid formula **I** esters will comprise various phospholipids. Phospholipid esters of the compounds of formula **I** will typically comprise a formula **I** compound esterified *via* its carbon 1 carboxylate to the *sn*-1 or *sn*-2 position alcohol, or both, of the glycerol backbone of the phospholipid. If the *sn*-1 or *sn*-2 position of the glyceryl ester class does not contain an ester of a compound of formula **I**, then such carbon positions of the glycerol backbone will comprise a methylene, ether or ester moiety linked to a substituted or unsubstituted $C_{12-30}$ alkyl or alkenyl (the alkenyl group containing one or more double bonds); alkyl(cycloalkyl)alkyl; alkyl(cycloalkyl); alkyl(heteroaryl); alkyl(heteroaryl)alkyl; or alkyl-M-Q; wherein the substitution is alkyl, halo, hydroxy, or functionally modified hydroxy; M is O or S; and Q is H, alkyl, alkyl(cycloalkyl)alkyl, alkyl(cycloalkyl), alkyl(heteroaryl) or alkyl(heteroaryl)alkyl. However, at least one of the *sn*-1 or *sn*-2 position alcohols of the glycerol backbone must form an ester with a compound of formula **I** *via* the carbon 1 carboxylate of the latter. Preferred phospholipid-formula **I**) esters will be of the phosphatidylethanolamine, phosphatidylcholine, phosphatidylserine, and phospatidylinositol type. The most preferred phospholipid-formula **I** esters will comprise a formula **I** compound esterified *via* its carbon 1 carboxylate to the alcohol at the *sn*-2 position of phosphatidylcholine, phosphatidylethanolamine or phosphatidylinositol. The phospholipid-formula **I** esters and sphingomyelin amides may be synthesized using various phospholipid synthetic methods known in the art; see for example, Tsai *et al.*, Biochemistry, volume 27,

page 4619 (1988); and Dennis *et al*., Biochemistry, volume 32, page 10185 (1993).

Included within the scope of the present invention are the individual enantiomers of the title compounds, as well as their racemic and non-racemic mixtures. The individual enantiomers can be enantioselectively synthesized from the appropriate enantiomerically pure or enriched starting material by means such as those described below. Alternatively, they may be enantioselectively synthesized from racemic/non-racemic or achiral starting materials. (*Asymmetric Synthesis;* J. D. Morrison and J. W. Scott, Eds.; Academic Press Publishers: New York, 1983-1985, volumes 1-5; *Principles of Asymmetric Synthesis;* R.E. Gawley and J. Aube, Eds.; Elsevier Publishers: Amsterdam, 1996). They may also be isolated from racemic and non-racemic mixtures by a number of known methods, e.g. by purification of a sample by chiral HPLC (*A Practical Guide to Chiral Separations by HPLC;* G. Subramanian, Ed.; VCH Publishers: New York, 1994; *Chiral Separations by HPLC;* A.M. Krstulovic, Ed.; Ellis Horwood Ltd. Publishers, 1989), or by enantioselective hydrolysis of a carboxylic acid ester sample by an enzyme (Ohno, M.; Otsuka, M. Organic Reactions, volume 37, page 1 (1989)). Those skilled in the art will appreciate that racemic and non-racemic mixtures may be obtained by several means, including without limitation, nonenantioselective synthesis, partial resolution, or even mixing samples having different enantiomeric ratios. Also included within the scope of the present invention are the individual isomers substantially free of their respective enantiomers.

As used herein, the terms "pharmaceutically acceptable salt" and "pharmaceutically acceptable ester" means any salt or ester, respectively, that would be suitable for therapeutic administration to a patient by any conventional means without significant deleterious health consequences; and "ophthalmically acceptable salt" and "ophthalmically acceptable ester" means any pharmaceutically acceptable salt or ester, respectively, that would be suitable for ophthalmic application, *i.e.* nontoxic and non-irritating.

The term "free hydroxy group" means an OH. The term "functionally modified hydroxy group" means an OH which has been functionalized to form: an ether, in which an alkyl, aryl, cycloalkyl, heterocycloalkyl, alkenyl, cycloalkenyl, heterocycloalkenyl, alkynyl, or heteroaryl group is substituted for the hydrogen; an ester, in which an acyl group is substituted for the hydrogen; a carbamate, in which an aminocarbonyl group is substituted for the hydrogen; or a carbonate, in which an aryloxy-, heteroaryloxy-, alkoxy-, cycloalkoxy-, heterocycloalkoxy-, alkenyloxy-, cycloalkenyloxy-, heterocycloalkenyloxy-, or alkynyloxy-carbonyl group is substituted for the hydrogen. Preferred moieties include OH, $OCH_2C(O)CH_3$,$OCH_2C(O)C_2H_5$, $OCH_3$, $OCH_2CH_3$, $OC(O)CH_3$, and $OC(O)C_2H_5$.

The term "free amino group" means an $NH_2$. The term "functionally modified amino group" means an $NH_2$ which has been functionalized to form: an aryloxy-, heteroaryloxy-, alkoxy-, cycloalkoxy-, heterocycloalkoxy-, alkenyl-, cycloalkenyl-, heterocycloalkenyl-, alkynyl-, or hydroxy-amino group, wherein the appropriate group is substituted for one of the hydrogens; an aryl-, heteroaryl-, alkyl-, cycloalkyl-, heterocycloalkyl-, alkenyl-, cycloalkenyl-, heterocycloalkenyl-, or alkynyl-amino group, wherein the appropriate group is substituted for one or both of the hydrogens; an amide, in which an acyl group is substituted for one of the hydrogens; a carbamate, in which an aryloxy-, heteroaryloxy-, alkoxy-, cycloalkoxy-, heterocycloalkoxy-, alkenyl-, cycloalkenyl-, heterocycloalkenyl-, or alkynyl-carbonyl group is substituted for one of the hydrogens; or a urea, in which an aminocarbonyl group is substituted for one of the hydrogens. Combinations of these substitution patterns, for example an $NH_2$ in which one of the hydrogens is replaced by an alkyl group and the other hydrogen is replaced by an alkoxycarbonyl group, also fall under the definition of a functionally modified amino group and are included within the scope of the present invention. Preferred moieties include $NH_2$, $NHCH_3$, $NHC_2H_5$, $N(CH_3)_2$, $NHC(O)CH_3$, NHOH, and $NH(OCH_3)$.

The term "free thiol group" means an SH. The term "functionally modified thiol group" means an SH which has been functionalized to form: a thioether, where an alkyl, aryl, cycloalkyl, heterocycloalkyl, alkenyl, cycloalkenyl, heterocycloalkenyl, alkynyl, or heteroaryl group is substituted for the hydrogen; or a thioester, in which an acyl group is substituted for the hydrogen. Preferred moieties include SH, $SC(O)CH_3$, $SCH_3$, $SC_2H_5$, $SCH_2C(O)C_2H_5$, and $SCH_2C(O)CH_3$.

The term "acyl" represents a group that is linked by a carbon atom that has a double bond to an oxygen atom and a single bond to another carbon atom.

The term "alkyl" includes straight or branched chain aliphatic hydrocarbon groups that are saturated and have 1 to 15 carbon atoms. The alkyl groups may be interrupted by one or more heteroatoms, such as oxygen, nitrogen, or sulfur, and may be substituted with other groups, such as halogen, hydroxyl, aryl, cycloalkyl, aryloxy, or alkoxy. Preferred straight or branched alkyl groups include methyl, ethyl, propyl, isopropyl, butyl and *t*-butyl.

The term "cycloalkyl" includes straight or branched chain, saturated or unsaturated aliphatic hydrocarbon groups which connect to form one or more rings, which can be fused or isolated. The rings may be substituted with other groups, such as halogen, hydroxyl, aryl, aryloxy, alkoxy, or lower alkyl. Preferred cycloalkyl groups include cyclopropyl, cyclobutyl, cyclopentyl and cyclohexyl.

The term "heterocycloalkyl" refers to cycloalkyl rings that contain at least one heteroatom such as O, S, or N in the ring, and can be fused or isolated. The rings may be substituted with other groups, such as halogen, hydroxyl, aryl, aryloxy, alkoxy, or lower alkyl. Preferred heterocycloalkyl groups include pyrrolidinyl, tetrahydrofuranyl, piperazinyl, and tetrahydropyranyl.

The term "alkenyl" includes straight or branched chain hydrocarbon groups having 1 to 15 carbon atoms with at least one carbon-carbon double bond, the chain being optionally interrupted by one or more heteroatoms. The chain hydrogens may be substituted with other groups, such as halogen. Preferred straight or branched alkeny groups include, allyl, 1-butenyl, 1-methyl-2-propenyl and 4-pentenyl.

The term "cycloalkenyl" includes straight or branched chain, saturated or unsaturated aliphatic hydrocarbon groups which connect to form one or more nonaromatic rings containing a carbon-carbon double bond, which can be fused or isolated. The rings may be substituted with other groups, such as halogen, hydroxyl, alkoxy, or lower alkyl. Preferred cycloalkenyl groups include cyclopentenyl and cyclohexenyl.

The term "heterocycloalkenyl" refers to cycloalkenyl rings which contain one or more heteroatoms such as O, N, or S in the ring, and can be fused or isolated.. The rings may be substituted with other groups, such as halogen, hydroxyl, aryl, aryloxy, alkoxy, or lower alkyl. Preferred heterocycloalkenyl groups include pyrrolidinyl, dihydropyranyl, and dihydrofuranyl.

The term "carbonyl group" represents a carbon atom double bonded to an oxygen atom, wherein the carbon atom has two free valencies.

The term "aminocarbonyl" represents a free or functionally modified amino group bonded from its nitrogen atom to the carbon atom of a carbonyl group, the carbonyl group itself being bonded to another atom through its carbon atom.

The term "lower alkyl" represents alkyl groups containing one to six carbons ($C_1$-$C_6$).

The term "halogen" represents fluoro, chloro, bromo, or iodo.

The term "aryl" refers to carbon-based rings which are aromatic. The rings may be isolated, such as phenyl, or fused, such as naphthyl. The ring hydrogens may be substituted with other groups, such as lower alkyl, halogen, free or functionalized hydroxy, trihalomethyl, *etc.* Preferred aryl groups include phenyl, 3-(trifluoromethyl)phenyl, 3-chlorophenyl, and 4-fluorophenyl.

The term "heteroaryl" refers to aromatic hydrocarbon rings which contain at least one heteroatom such as O, S, or N in the ring. Heteroaryl rings may be isolated, with 5 to 6 ring atoms, or fused, with 8 to 10 atoms. The heteroaryl ring(s) hydrogens or heteroatoms with open valency may be substituted with other groups, such as lower alkyl or halogen. Examples of heteroaryl groups include imidazole, pyridine, indole, quinoline, furan, thiophene, pyrrole, tetrahydroquinoline, dihydrobenzofuran, and dihydrobenzindole.

The terms "aryloxy", "heteroaryloxy", "alkoxy", "cycloalkoxy", "heterocycloalkoxy", "alkenyloxy", "cycloalkenyloxy", "heterocycloalkenyloxy", and "alkynyloxy" represent an aryl, heteroaryl, alkyl, cycloalkyl, heterocycloalkyl, alkenyl, cycloalkenyl, heterocycloalkenyl, or alkynyl group attached through an oxygen linkage.

The terms "alkoxycarbonyl", "aryloxycarbonyl", "heteroaryloxycarbonyl", "cycloalkoxycarbonyl", "heterocycloalkoxycarbonyl", "alkenyloxycarbonyl", "cycloalkenyloxycarbonyl", "heterocycloalkenyloxycarbonyl", and "alkynyloxycarbonyl" represent an alkoxy, aryloxy, heteroaryloxy, cycloalkoxy, heterocycloalkoxy, alkenyloxy, cycloalkenyloxy, heterocycloalkenyloxy, or alkynyloxy group bonded from its oxygen atom to the carbon of a carbonyl group, the carbonyl group itself being bonded to another atom through its carbon atom.

Preferred compounds of the present invention include those of formula **I**, wherein:

$R^1$ is $CO_2R$, where R is H or an ophthalmically acceptable cationic salt, or $CO_2R$ forms an ophthalmically acceptable ester moiety;
A is $L_1$-$A_2$-$L_2$;
B is C≡C or *cis*-CH=CH and D is C≡C or *trans*-CH=CH;
K is *cis*-CH=CH; and
Y is

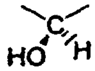

or

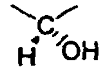

Other preferred compounds of the present invention include those of formula **I**, wherein:

$R^1$ is $CO_2R$, where R is H or an ophthalmically acceptable cationic salt, or $CO_2R$ forms an ophthalmically acceptable ester moiety;
A is $L_3$-$A_2$-$L_4$;
$L_3$ is *trans*-$CH_2CH$=CH, *trans*-CH=$CHCH_2$, or $CH_2C$≡C;
X is *trans*-$CH_2CH_2CH$=CH, $CH_2CH_2C$≡C, *trans*-$CH_2CH$=$CHCH_2$, or *trans*-CH=$CHCH_2CH_2$; and

Y is

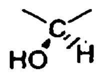

or

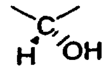

Still other preferred compounds of the present invention include those of formula **I**, wherein:

$R^1$ is $CO_2R$, where R is H or an ophthalmically acceptable cationic salt, or $CO_2R$ forms an ophthalmically acceptable ester moiety;
A is $L_5$-$A_2$-$L_3$;
$L_3$ is *trans*-$CH_2CH{=}CH$, *trans*-$CH{=}CHCH_2$, or $CH_2C{\equiv}C$;
B is *cis*-CH=CH or C≡C;
D is *trans*-CH=CH or C≡C; and
Y is

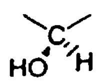

or

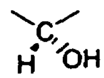

Among the especially preferred of the foregoing compounds are those whose preparations are detailed in the following examples 2,3,5,6,8-10 and 12-19.

The other examples are reference examples which exemplify the production of HETE-derivatives.

**Reference Example 1:**

Synthesis of 1

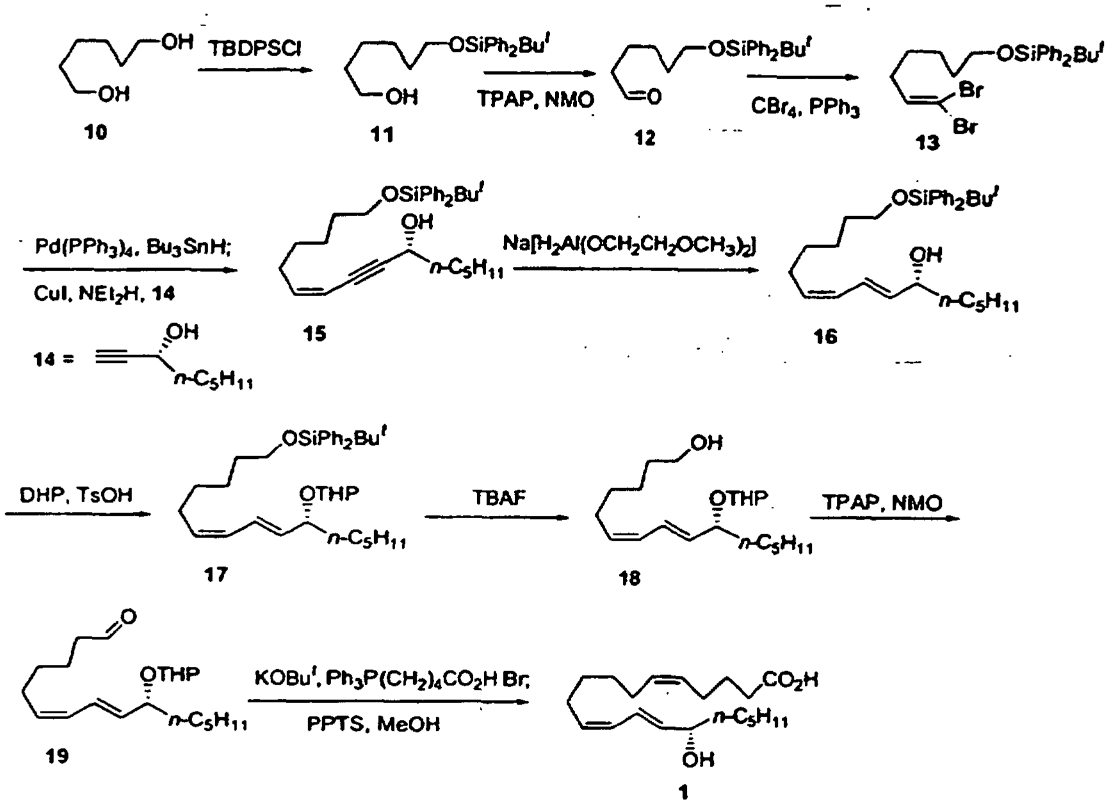


Compound **1**

Treatment of 1,6-hexanediol (**10**) with 0.9 equivalents of *t*-butylchlorodiphenylsilane (TBDPSCl) in the presence of imidazole and 4-(dimethylamino)pyridine (DMAP) in *N,N*-dimethylformamide (DMF) affords monosilyl ether **11,** which is oxidized with stoichiometric *N*-methylmorpholine *N*-oxide (NMO) in the presence of a catalytic amount of tetra-*n*-propylammonium perruthenate (TPAP) to provide aldehyde **12.** Dibromoolefination of **12** using $CBr_4$ and $PPh_3$ gives **13.** Conversion of **13** to enynol **15** is accomplished in two steps: first, treatment of **13** with 1 equivalent of $Bu_3SnH$ in toluene in the presence of a catalytic amount of $Pd(PPh_3)_4$ to afford the corresponding *cis*-vinyl bromide, followed by addition of CuI, $HNEt_2$, and chiral enantiopure propargyl alcohol **14** [for the preparation of **14,** see: Midland *et. al.*, Tetrahedron, 40:1371 (1984)]. Reduction of **15** with $Na[H_2Al(OCH_2CH_2OCH_3)_2]$ affords diene **16,** which is treated with 3,4-dihydro-2*H*-pyran (DHP) and a catalytic amount of *p*-toluenesulfonic acid monohydrate (TsOH) to give ether **17**. Desilylation of **17** with tetra-*n*-butylammonium fluoride (TBAF) yields alcohol **18,** which is oxidized with TPAP/NMO to provide aldehyde **19.** Condensation of **19** with $Ph_3P(CH_2)_4CO_2H$ Br in the presence of $KOBu^t$, followed by treatment of the resultant eneacid with pyridinium *p*-toluenesulfonate (PPTS) in warm methanol, affords **1**.

Example 2:

### Preparation of 2α and 2β

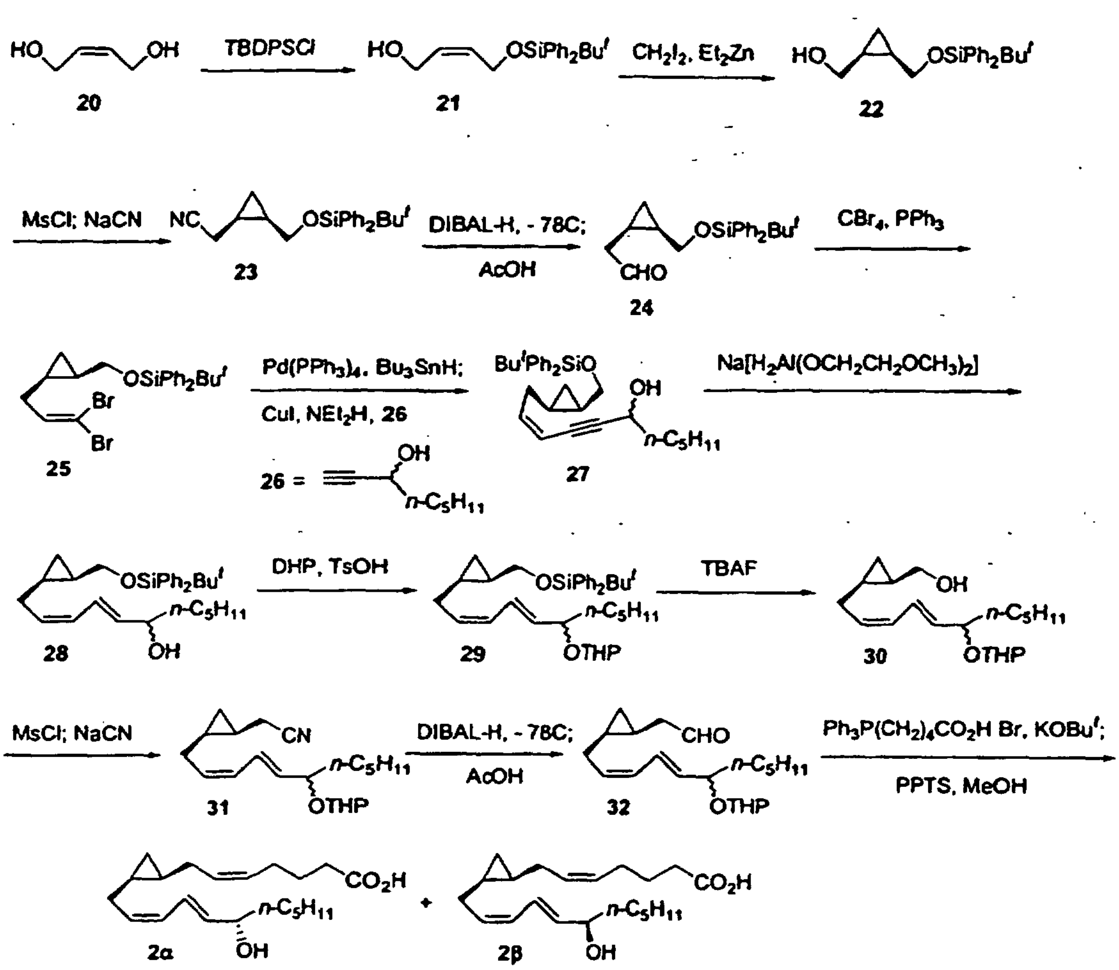


Compounds **2**α and **2**β

Monosilylation of (2*Z*)-2-buten-1,4-diol (**20**) with TBDPSCI provides silyl ether **21,** which is reacted with diiodomethane and diethylzinc to afford cyclopropane **22.** Sequential reaction with mesyl chloride and NaCN provides nitrile **23. 23** is reduced with diisobutylaluminum hydride (DIBAL-H) at low temperature, and the intermediate imine is hydrolyzed with aqueous acetic acid to afford aldehyde **24.** Condensation of **24** with $CBr_4$ and $PPh_3$ gives dibromoolefin **25.** Monoreduction of **25** using stoichiometric $Bu_3SnH$ and catalytic $Pd(PPh_3)_4$ affords an intermediate *Z*-vinyl bromide, which in the same pot is reacted with 1-octyn-3-ol (commercially available from Aldrich Chemical Co., Milwaukee, WI) in the presence of CuI and diethylamine to provide enyne **27.** Reduction of **27** with sodium bis(2-methoxyethyoxy) aluminum hydride yields *Z*, *E*- dienyl alcohol **28,** which is converted THP ether **29** using DHP and TsOH. Desilylation of **29** with TBAF affords alcohol **30,** which is extended to cyanide **31** by sequential treatment with mesyl chloride and NaCN. Conversion to aldehyde **32** effected by reduction with DIBAL-H at -78 °C, followed by hydrolysis of the resulting metalloenamine with aqueous acetic acid at 0 °C. Wittig condensation with $Ph_3P(CH_2)_4CO_2H$ Br in the presence of $KOBu^t$, followed by deprotection of the resultant eneacid with PPTS in MeOH, yields **2**α and **2**β after separation of the two C-15 diastereomers using silica gel chromatography.

Example 3:

Synthesis of $3\alpha$ and $3\beta$

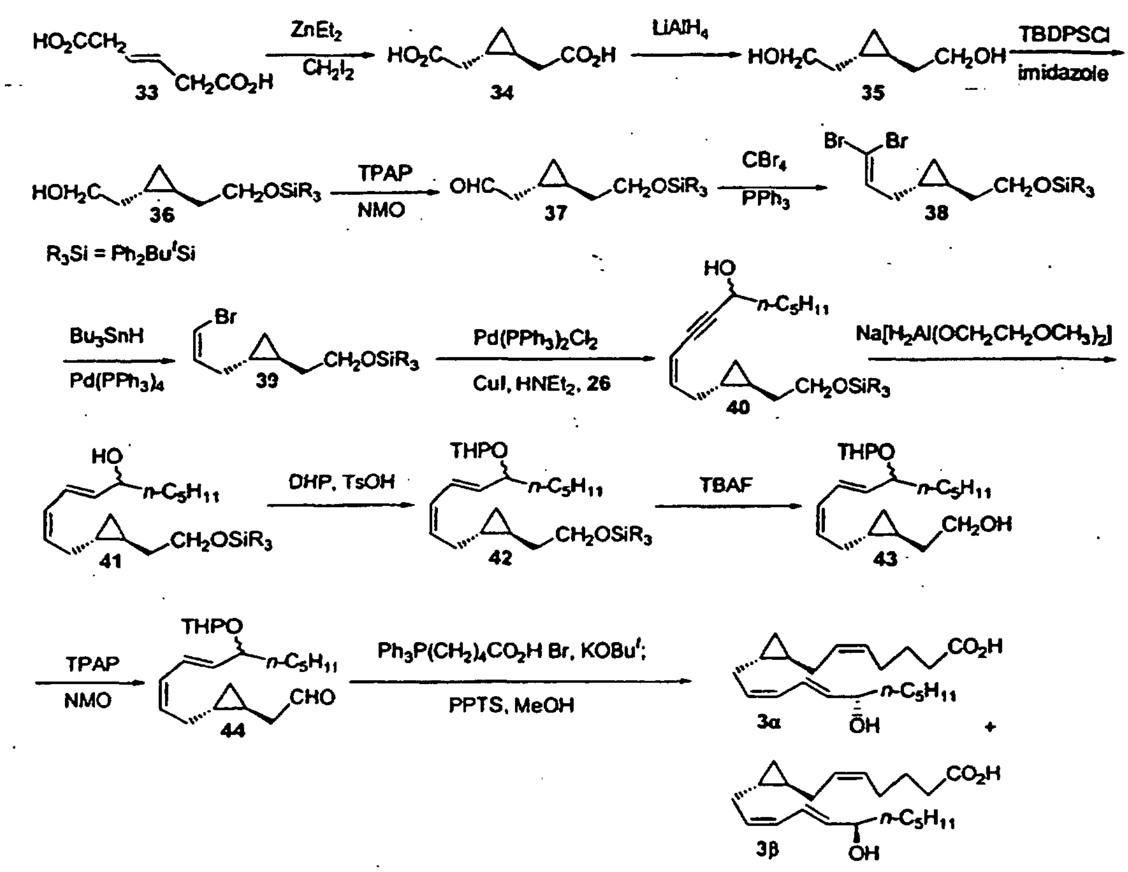


Compounds $3\alpha$ and $3\beta$

Treatment of *trans*-β-hydromuconic acid (**33**) with diethyl zinc and diiodomethane affords cyclopropane **34**, which is reduced to diol **35** with $LiAlH_4$. Monosilylation with TBDPSCl provides silyl ether **36**, which is oxidized to aldehyde **37** using TPAP/NMO. Reaction of **37** with $CBr_4$ and $PPh_3$ gives dibromoolefin **38,** which is converted to *Z*-vinyl bromide **39** using stoichiometric $Bu_3SnH$ in the presence of catalytic $Pd(PPh_3)_4$. Sonogishira coupling of **39** with 1-octyn-3-ol in the presence of CuI, $Pd(PPh_3)_2Cl_2$, and $HNEt_2$ yields enyne **40,** which is reduced to the corresponding *E*-allyl alcohol **41** with $Na[H_2Al(CH_2CH_2OCH_3)_2]$. Treatment of **41** with DHP and TsOH affords THP ether **42,** which is desilylated to alcohol **43** with TBAF in THF. Oxidation of **43** to aldehyde **44** is achieved using TPAP/NMO. This aldehyde is reacted with $Ph_3P(CH_2)_4CO_2H$ Br in the presence of $KOBu^t$, and the intermediate eneacid is deprotected with PPTS in MeOH to afford targets $3\alpha$ and $3\beta$ after separation of the two C-15 diastereomers using silica gel chromatography.

Reference Example 4:

Synthesis of **4**

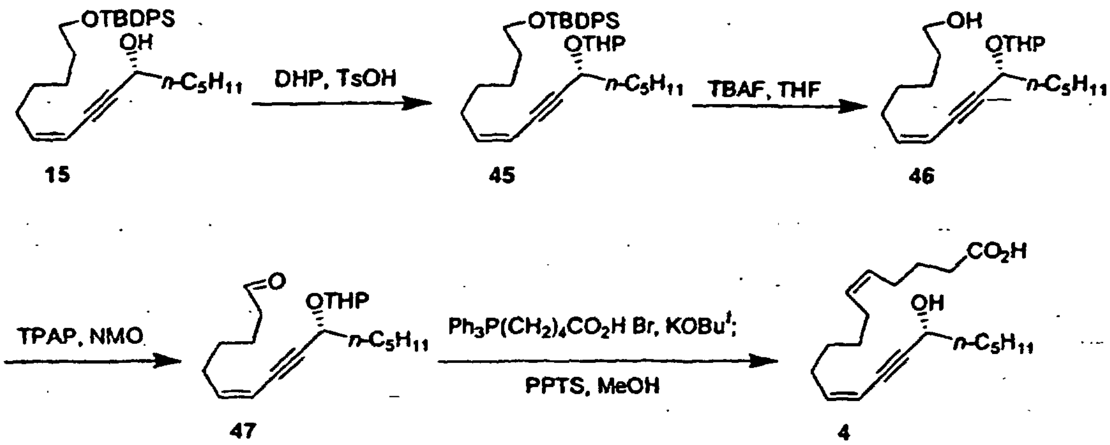


15 45 46 47 4

Compound **4**

Alcohol **15** is protected as its THP ether **45** by treatment with DHP and TsOH. Desilylation of **45** with TBAF in THF provides alcohol **46,** which is oxidized to aldehyde **47** with TPAP and NMO. Wittig reaction of **47** with $Ph_3P(CH_2)_4CO_2H$ Br in the presence of $KOBu^t$ affords an intermediate eneacid, which is deprotected to **4** using PPTS in MeOH.

Example 5:

Preparation of 5α and 5β

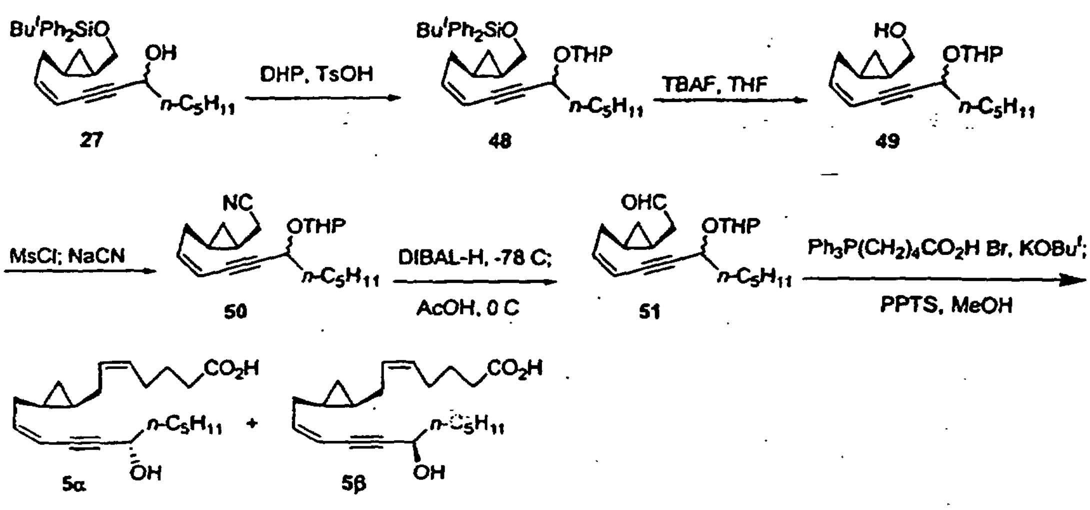


27 48 49 50 51 5α + 5β

Compounds **5**α and **5**β

Treatment of enynol **27** with DHP and TsOH affords THP ether **48,** which is desilylated using TBAF in THF to afford alcohol **49.** Sequential treatment of **49** with mesyl chloride and then NaCN provides nitrile **50,** which is reduced to aldehyde **51** by reaction with DIBAL-H at -78 °C and acetic acid at 0 °C. Wittig condensation of **51** with $Ph_3P(CH_2)_4CO_2H$ Br in the presence of $KOBu^t$, followed by treatment of the intemediate eneacid with PPTS in MeOH, gives **5**α and **5**β after separation of the two C-15 diastereomers using silica gel chromatography.

**Example 6:**

**Synthesis of 6**

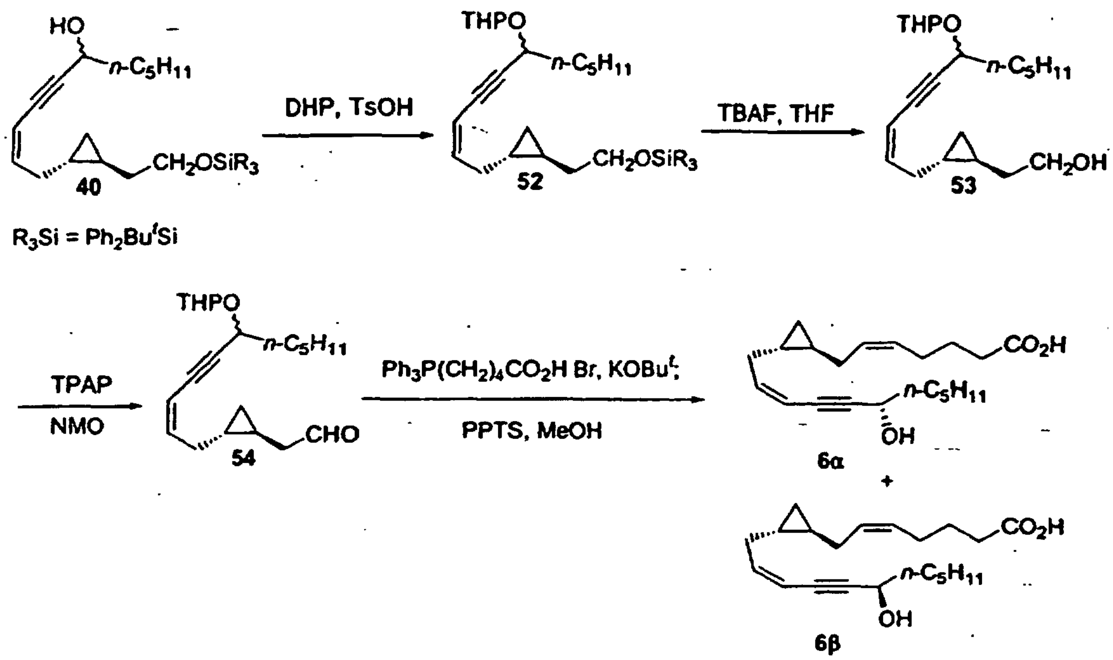


Compounds **6**α and **6**β

Reaction of enynol **40** with DHP and TsOH affords THP ether **52,** which is desilylated using TBAF in THF to afford alcohol **53.** Oxidation of **53** using TPAP and NMO provides aldehyde **54,** which undergoes Wittig condensation with $Ph_3P(CH_2)_4CO_2H$ Br/$KOBu^t$ and deprotection with PPTS in MeOH to give compounds **6**α and **6**β after separation of the two C-15 diastereomers using silica gel chromatography.

Reference Example 7:

Synthesis of **7**

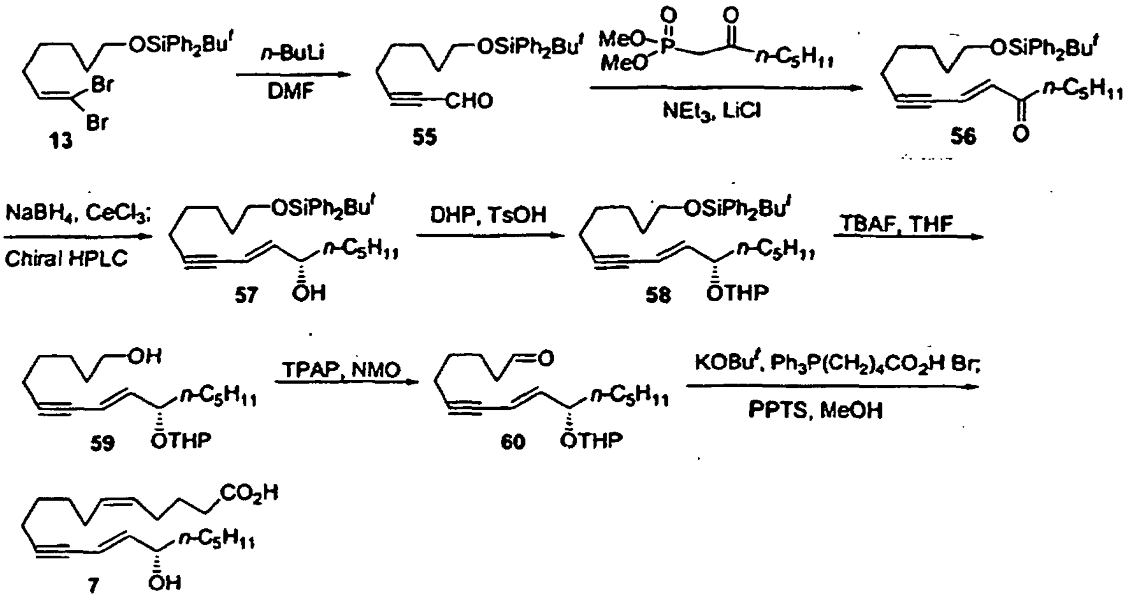


Compound **7**

Treatment of dibromoolefin **13** with *n*-BuLi and N,N-dimethylformamide affords ynal **55,** which is condensed with dimethyl (2-oxoheptyl)phosphonate in the presence of $NEt_3$ and LiCl to provide enone **56. 56** is reduced to 15*S*-alcohol **57** by treatment with $NaBH_4$ and $CeCl_3$, followed by separation of the resulting racemic mixture using HPLC with a chiral stationary phase. Treatment of **57** with DHP and TsOH gives THP ether **58,** which is desilylated with TBAF in THF to yield alcohol **59.** Oxidation of **59** with TPAP and NMO affords aldehyde **60. 60** is treated with $Ph_3P(CH_2)_4CO_2H$ Br in the presence of $KOBu^t$, followed by PPTS in MeOH, to give **7**.

Example 8:

Preparation of **8α and 8β**

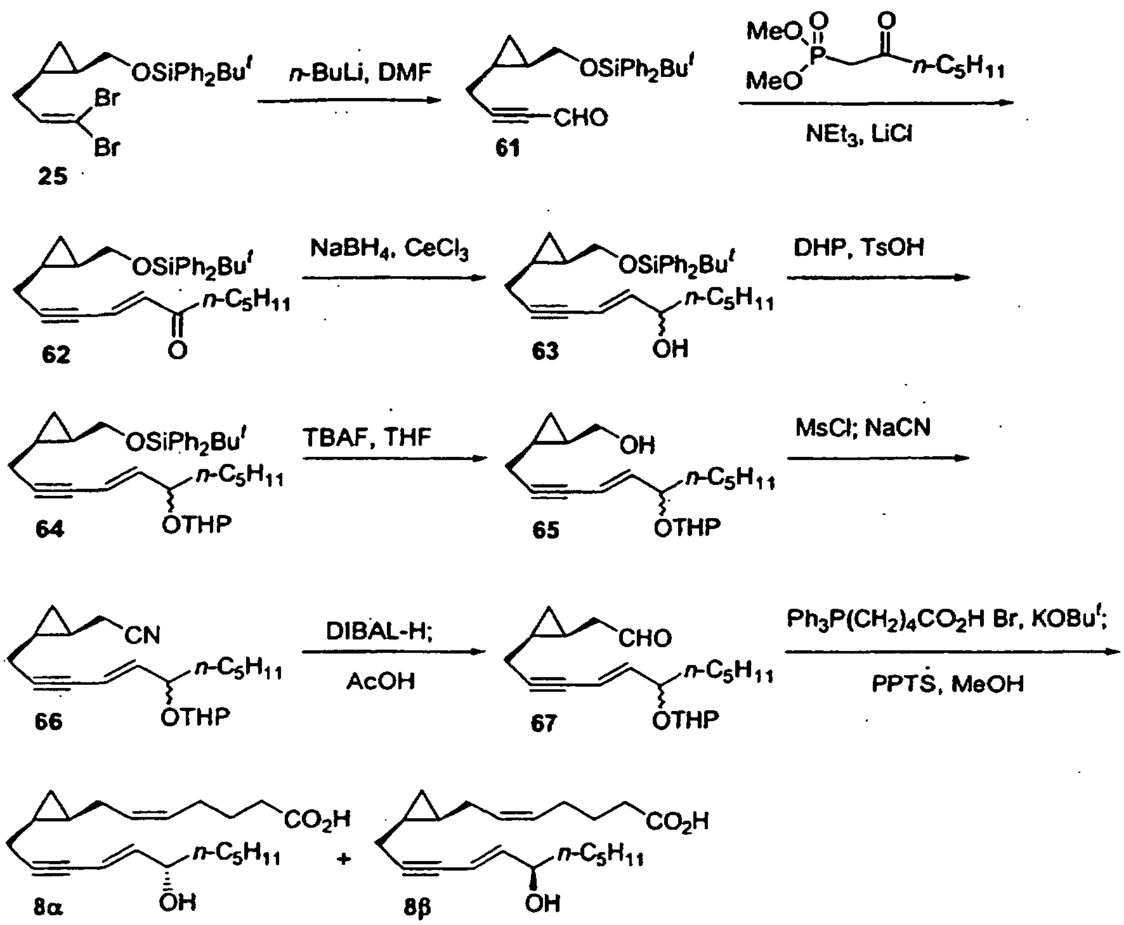


**25** **61** **62** **63** **64** **65** **66** **67** **8α** + **8β**

Compounds **8a** and **8**β

Treatment of dibromoolefin **25** with *n*-BuLi and N,N-dimethylformamide affords ynal **61,** which is condensed with dimethyl (2-oxoheptyl)phosphonate in the presence of $NEt_3$ and LiCl to provide enone **62. 62** is reduced to 15*R*, *S*-alcohol **63** by treatment with $NaBH_4$ and $CeCl_3$. Treatment of **63** with DHP and TsOH gives THP ether **64,** which is desilylated with TBAF in THF to yield alcohol **65.** Sequential treatment of **65** with mesyl chloride and then NaCN affords nitrile **66. 66** is converted to aldehyde **67** by reduction with DIBAL-H at -78 °C, followed by hydrolysis acetic acid at 0 °C. **67** is treated with $Ph_3P(CH_2)_4CO_2H$ Br in the presence of $KOBu^t$, followed by PPTS in MeOH, to give targets **8**α and **8**β after separation of the two C-15 diastereomers using silica gel chromatography.

**Example 9:**

**Preparation of 9α and 9β**

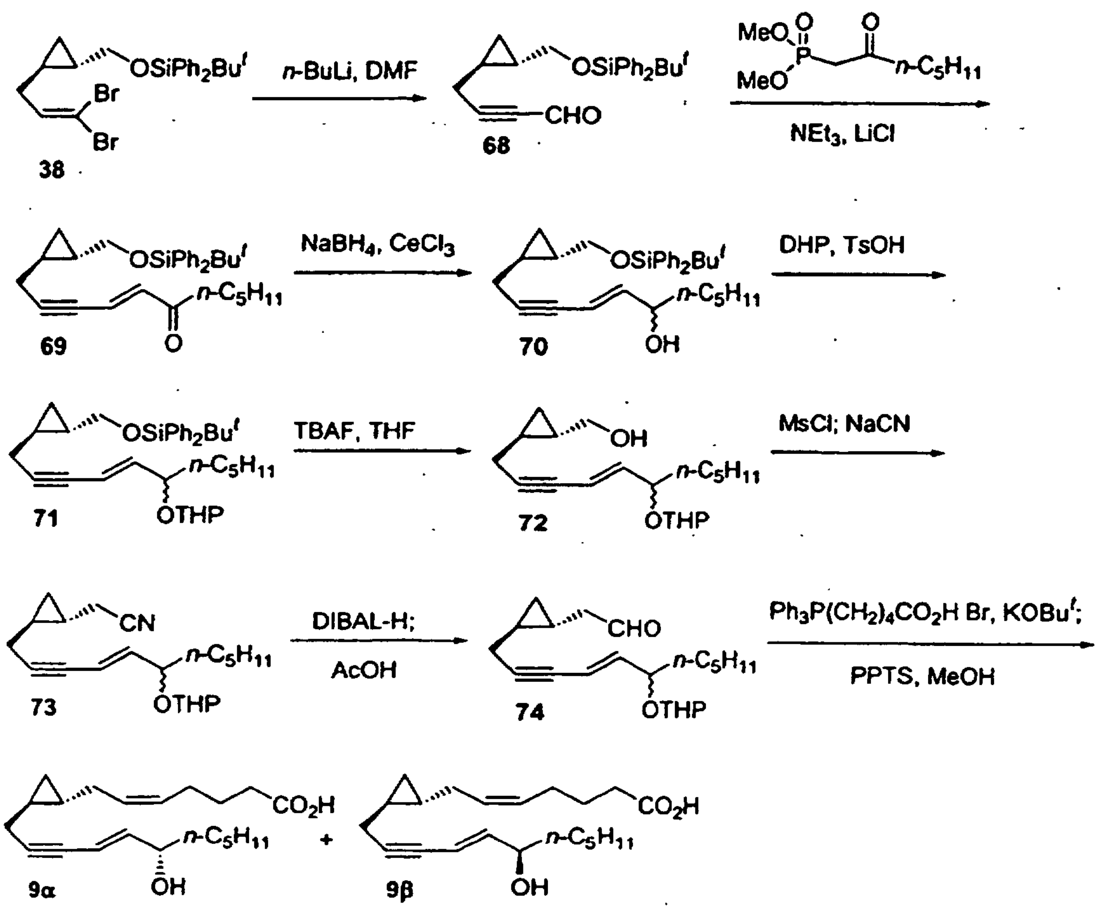


38, 68, 69, 70, 71, 72, 73, 74, 9α + 9β

Compounds **9**α and **9**β

Treatment of dibromoolefin **38** with *n*-BuLi and N,N-dimethylformamide affords ynal **68,** which is condensed with dimethyl (2-oxoheptyl)phosphonate in the presence of $NEt_3$ and LiCl to provide enone **69. 69** is reduced to 15*R*,*S*-alcohol **70** by treatment with $NaBH_4$ and $CeCl_3$. Treatment of **70** with DHP and TsOH gives THP ether **71**, which is desilylated with TBAF in THF to yield alcohol **72.** Sequential treatment of **72** with mesyl chloride and then NaCN affords nitrile **73. 73** is converted to aldehyde **74** by reduction with DIBAL-H at -78 °C, followed by hydrolysis acetic acid at 0 °C. **74** is treated with $Ph_3P(CH_2)_4CO_2H$ Br in the presence of $KOBu^t$, followed by PPTS in MeOH, to give compounds **9**α and **9**β after separation of the two C-15 diastereomers using silica gel chromatography.

**Example 10:**

Preparation of 75 and 76

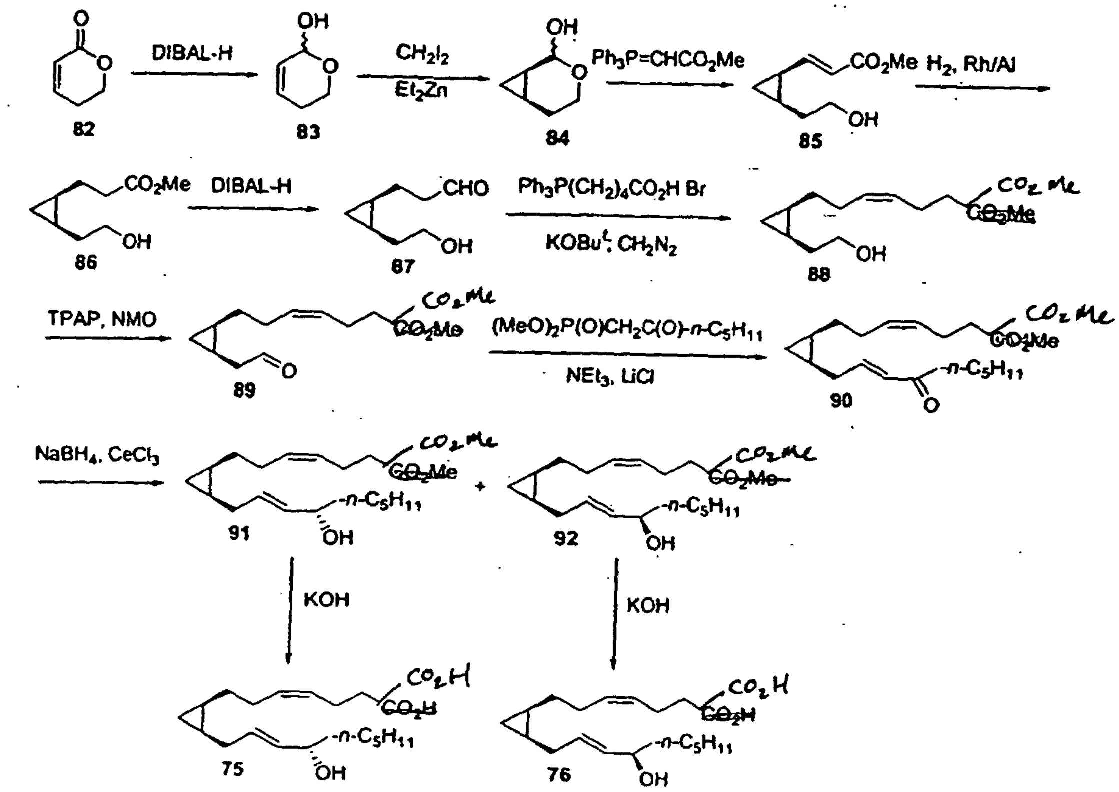


Compounds **75** and **76**

Reduction of commercially available dihydropyranone **82** with diisobutylaluminum hydride (DIBAL-H) affords lactol **83,** which is cyclopropanated with $CH_2I_2/Et_2Zn$ to provide **84. 84** is condensed with $Ph_3P{=}CHCO_2CH_3$ to yield enoate **85,** which is reduced under 1 atmosphere of hydrogen using Rhodium on Alumina catalyst to give **86. 86** is converted to aldehyde **87** by reduction with DIBAL-H. Condensation of **87** with $Ph_3P(CH_2)_4CO_2H$ Br in the presence of potassium *t*-butoxide (KOBu$^t$), followed by treatment of the intermediate acid with diazomethane, provides olefin **88.** Oxidation of **88** using catalytic tetra-*n*-propylammonium perruthenate (TPAP) and stoichiometric *N*-methylmorpholine *N*-oxide (NMO) gives aldehyde **89,** which is condensed with dimethyl (2-oxoheptyl)phosphonate in the presence of $NEt_3$ and LiCl to provide enone **90.** Treatment of **90** with $NaBH_4$ in the presence of $CeCl_3$ affords a mixture of two stereoisomeric alcohols, **91** and **92,** which are separated using silica gel chromatography. Treatment of the thus separated samples of **91** and **92** with KOH in MeOH/water affords the corresponding acids **75** and **76**.

**Reference Example 11:**

**Preparation of 77 and 78**

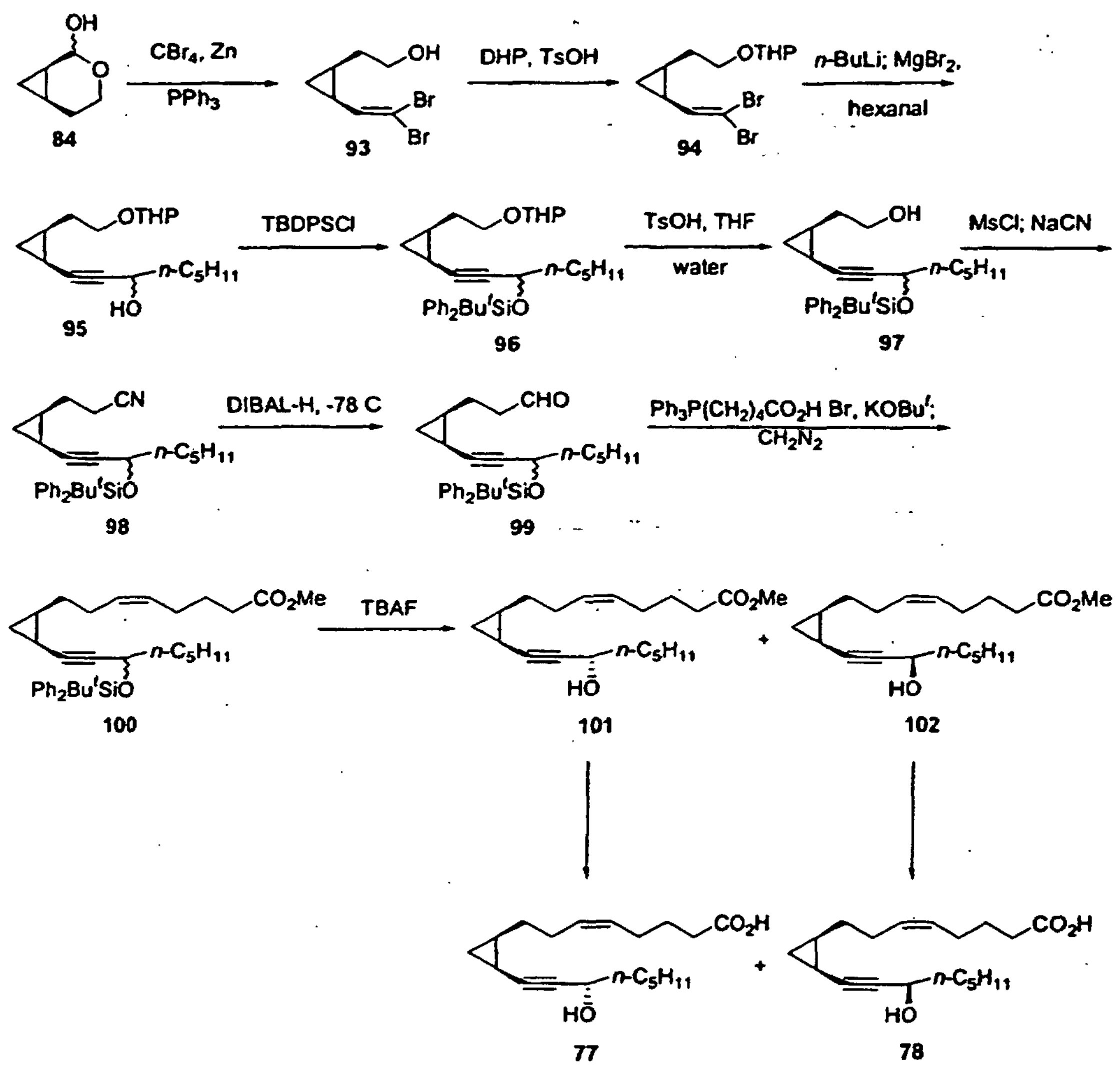


Compounds **77** and **78**

Reaction of lactol **84** with $CBr_4$, $PPh_3$, and Zn affords dibromoolefin **93,** which is protected as its THP ether **94** by treatment with 3,4-dihydro-2*H*-pyran (DHP) in the presence of catalyltic *p*-toluenesulfonic acid monohydrate (TsOH). Metallation of **94** with *n*-BuLi, transmetallation to the magnesium acetylide by addition of $MgBr_2$, and addition of hexanal affords alkynol **95**, which is protected as its *t*-butyldiphenylsilyl ether by treatment with *t*-butylchlorodiphenylsilane (TBDPSCl) and imidazole in the presence of catalytic 4-(dimethylamino)pyridine (DMAP). Removal of the THP protecting group from **95** is accomplished by treatment with TsOH in hot THF/water, to provide alcohol **96.** Sequential reaction of **96** with methanesulfonyl chloride (MsCl) in $CH_2Cl_2$ and then with NaCN in DMSO gives nitrile **97,** which is converted to aldehyde **98** by reaction with DIBAL-H at -78 °C, followed by acid hydrolysis with aqueous acetic acid at 0 °C. Wittig condensation of **98** with $Ph_3P(CH_2)_4CO_2H$ Br in the presence of KOBu$^t$, followed by esterification of the intermediate acid with diazomethane, affords olefin **99.** Treatment of **99** with tetra-*n*-butylammonium fluoride (TBAF) in THF, followed by chromatographic purification, gives the individual diastereomers **100** and **101**. These are converted to their respective free acids **77** and **78** by treatment with KOH in MeOH/water.

Example 12:

**Preparation of 79 and 80**

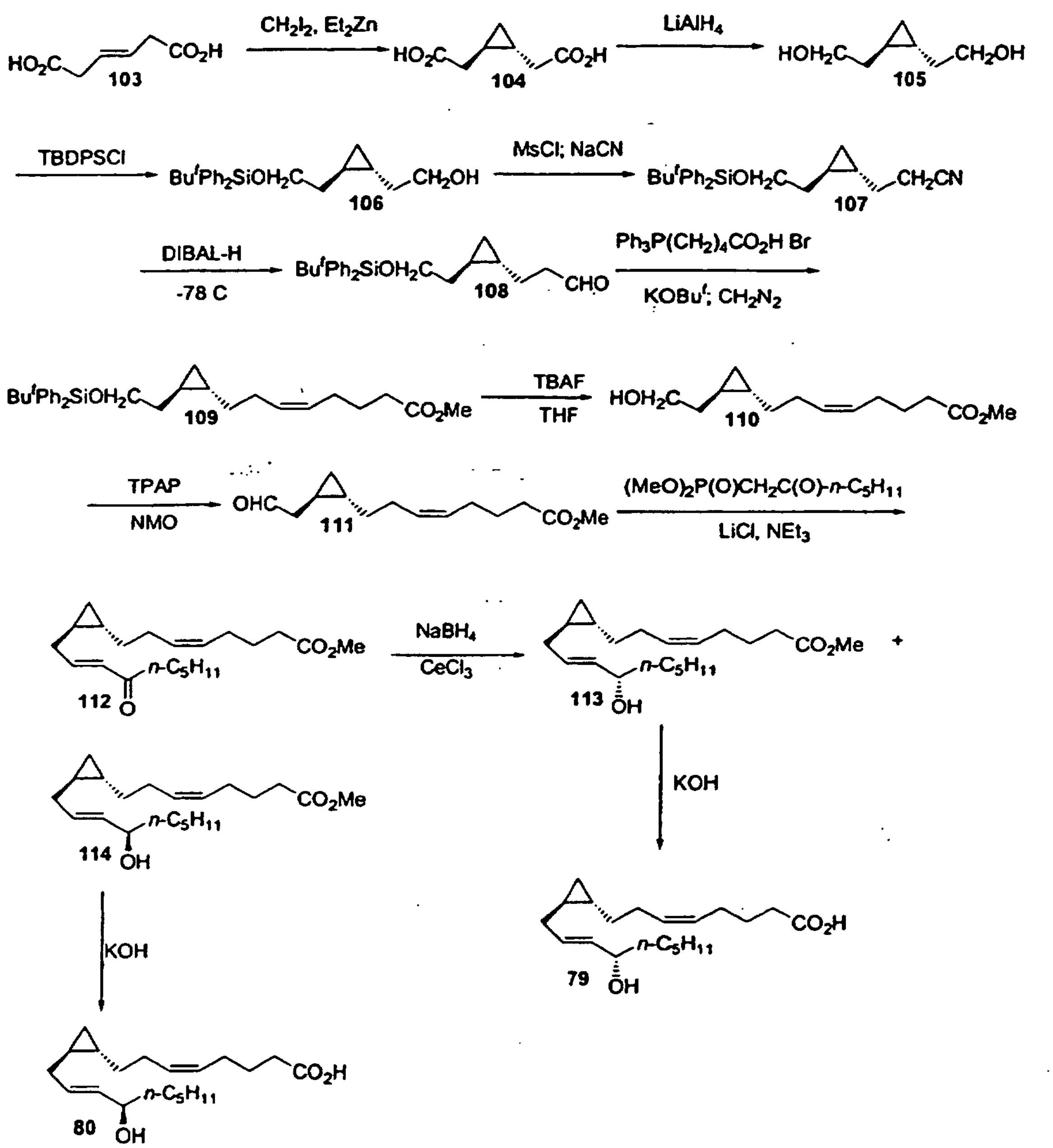


Compounds **79** and **80**

Treatment of *trans*-β-hydromuconic acid (103) with $CH_2I_2/Et_2Zn$ affords cyclopropane **104,** which is reduced to diol **105** with $LiAlH_4$. **105** is monosilylated with TBDPSCl in the presence of imidazole and DMAP provide silyl ether **106,** which is treated sequentially with MsCl in $CH_2Cl_2$ and then NaCN in DMSO to give nitrile **107. 107** is converted to aldehyde **108** by treatment with DIBAL-H at -78 °C followed by aqueous acetic acid at 0 °C. **108** is condensed with $Ph_3P(CH_2)_4CO_2H$ Br in the presence of $KOBu^t$, followed by esterification of the intermediate acid with diazomethane, to afford olefin **109. 109** is deprotected using TBAF in THF to give alcohol **119** which is oxidized using TPAP/NMO to yield aldehyde **111. 111** is condensed with dimethyl (2-oxoheptyl)phosphonate in the presence of LiCl and $NEt_3$ to

give enone **112,** which is reduced using $NaBH_4/CeCl_3$ to provide the α and β allyl alcohol diastereomers **113** and **114** after chromatographic purification. Saponification of each of these esters using KOH in aqueous methanol affords the acids **79** and **80** respectively.

## Example 13:

Preparation of **81** and **82**

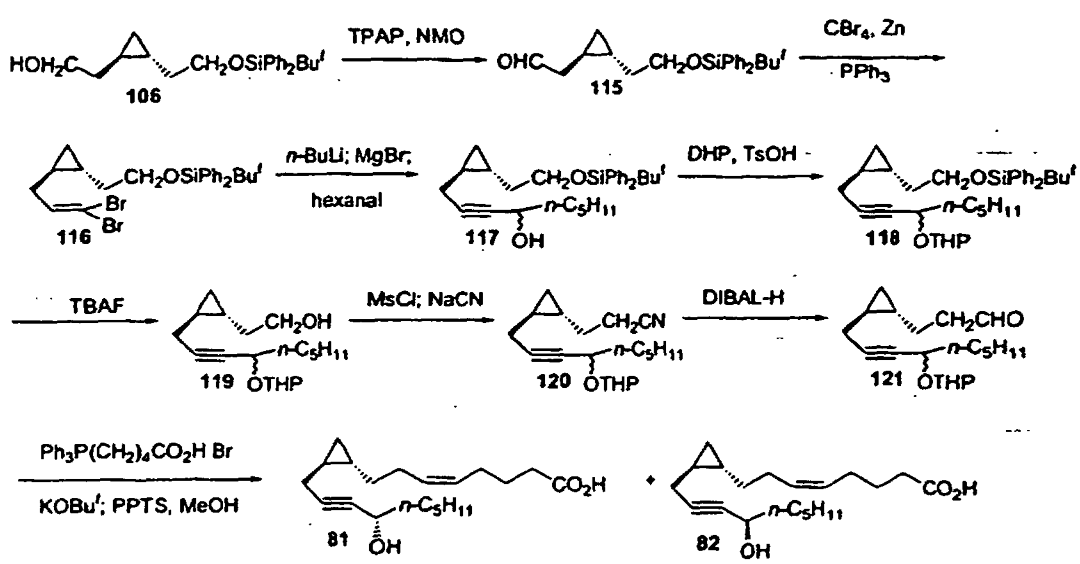


Compounds **81** and **82**

Alcohol **106** is oxidized using TPAP/NMO to give aldehyde **115,** which is condensed with $CBr_4$ in the presence of $PPh_3$ and Zn to afford dibromoolefin **116.** Treatment of **116** successively with *n*-BuLi, then $MgBr_2$, and finally hexanal affords ynol **117. 117** is protected as its THP ether by treatment with DHP and TsOH to give **118.** Desilylation of **118** with TBAF in THF affords alcohol **119,** which is treated sequentially with MsCl in $CH_2Cl_2$ and then NaCN in DMSO to yield nitrile **120.** Reduction of **120** with DIBAL-H at -78 °C, followed by hydrolysis with aqueous acetic acid at 0 °C, gives aldehyde **121.** Condensation of **121** with $Ph_3P(CH_2)_4CO_2H$ Br in the presence of $KOBu^t$, followed by treatment of the intermediate THP ether acid with pyridinium *p*-toluenesulfonate (PPTS) in warm methanol, affords the individual α and β propargyl alcohol diastereomeric acids **81** and **82** after chromatographic purification.

Example 14:

**Synthesis of 122 and 123**

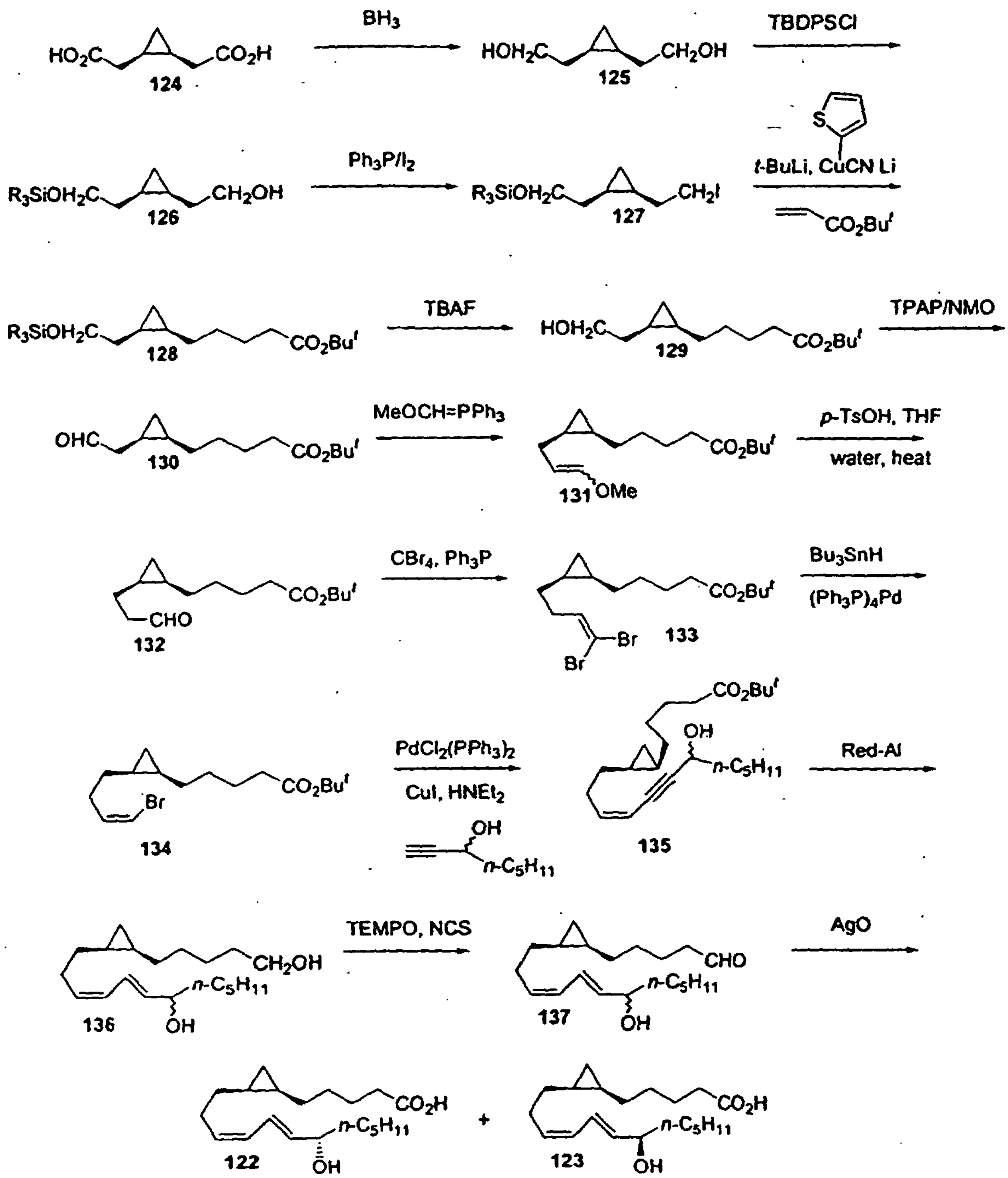


Compounds **122** and **123**

Reduction of diacid **124** (for the preparation of **13,** see: Neset *et. al., Tetrahedron* **1997**, *53*,10459, which is incorporated herein by reference) with $BH_3$ affords diol **125**, which is silylated with *t*-butyldiphenylsilyl chloride (TBDPSCl) in the presence of 4-(dimethylamino)pyridine (DMAP) and imidazole to afford silyl ether **126.** Treatment of **126** with $I_2$ and $PPh_3$ in toluene in the presence of imidazole affords iodide **127. 127** is treated sequentially with with *t*-butyllithium at -78 °C, lithium (2-thienyl)cyanocuprate, and *t*-butyl acrylate to afford the Michael adduct **128** after quenching with aqueous acid. **128** is desilylated to alcohol **129** using tetra-*n*-butylammonium fluoride (TBAF) in THF. Oxidation of **129** with catalytic tetra-*n*-propylammonium perrruthernate (TPAP) and stoichiometric *N*-methylmorpholine-*N*-oxide (NMO) provides aldehyde **130,** which is converted to enol ether **131** by Wittig reaction with $MeOCH{=}PPh_3$. Hydrolysis of **131** using catalytic *p*-toluenesulfonic acid monohydrate (TsOH) in THF/water with heating affords homologated aldehyde **132,** which is converted to dibromoolefin **133** by condensation with $CBr_4$ in the presence of $PPh_3$. Selective monoreduction of **133** with $Bu_3SnH$ in the presence of catalytic $Pd(PPh_3)_4$ gives *Z*-bromoalkene **134,** which upon treatment with 1-octyn-3-ol, CuI, and catalytic $PdCl_2(PPh_3)_2$ in $HNEt_2$ yields enynol **135. 135** is reduced with Na $[H_2Al(OCH_2CH_2OCH_3)_2]$ (Red-Al®) in toluene to provide diene diol **136**, which is selectively oxidized to hydroxyaldehyde **137** using catalytic 2,2,6,6-tetramethylpiperidinoxyl free radical (TEMPO) and stoichiometric *N*-chlorosuccinimide (NCS). Oxidation of **137** with silver (II) oxide, followed by chromatographic separation of the allyl alcohol diastereomers, affords the α isomer **122** and the β isomer **123**.

**Example 15:**

**Synthesis of 138 and 139**

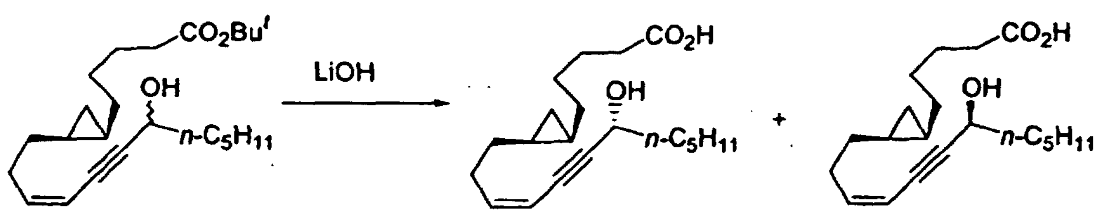


**135** **138** **139**

Compounds **138** and **139**

Saponification of ester **135** with LiOH in methanol/water, followed by chromatographic separation of the propargyl alcohol diastereomers, affords targets **138** and **139**.

Example 16:

**Synthesis of 144 and 145**

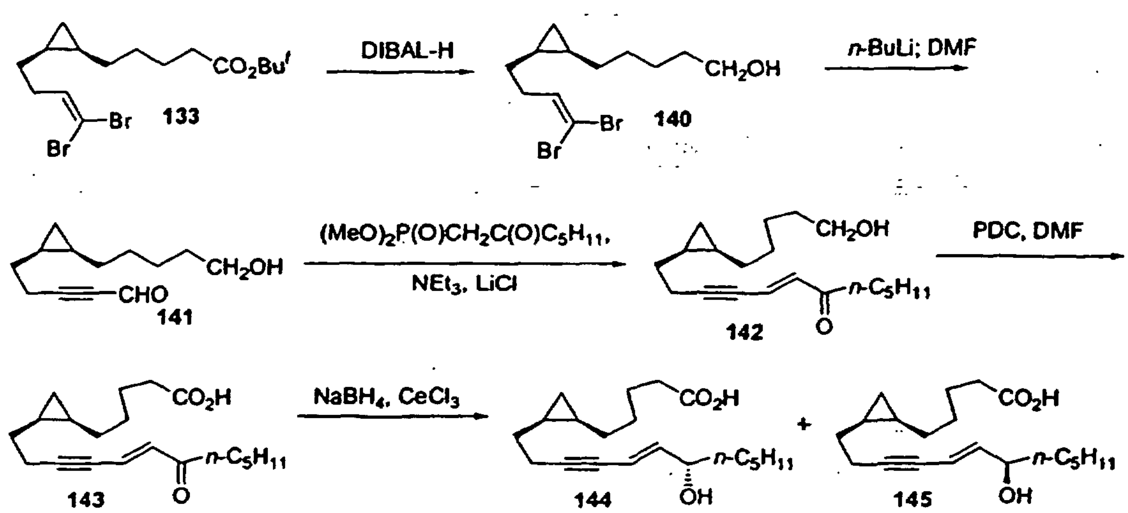


Compounds **144** and **145**

Reduction of ester **133** with diisobutylaluminum hydride (DIBAL-H) affords alcohol **140,** which is treated with three equivalents of *n*-butyllithium at -78 °C and then with *N*,*N*-dimethylformamide (DMF) to provide ynal **141**. Horner-Emmons condensation of **141** with dimethyl (2-oxoheptyl)phosphonate in the presence of LiCl and $NEt_3$ gives ynenone **142,** which is oxidized to acid **143** using pyridinium dichromate (PDC) in DMF. Reduction of **143** using $NaBH_4$ in the presence of $CeCl_3$, followed by chromatographic separation of the two allyl alcohol diastereomers, affords compounds **144** and **145**.

Example 17:

**Synthesis of160 and 161**

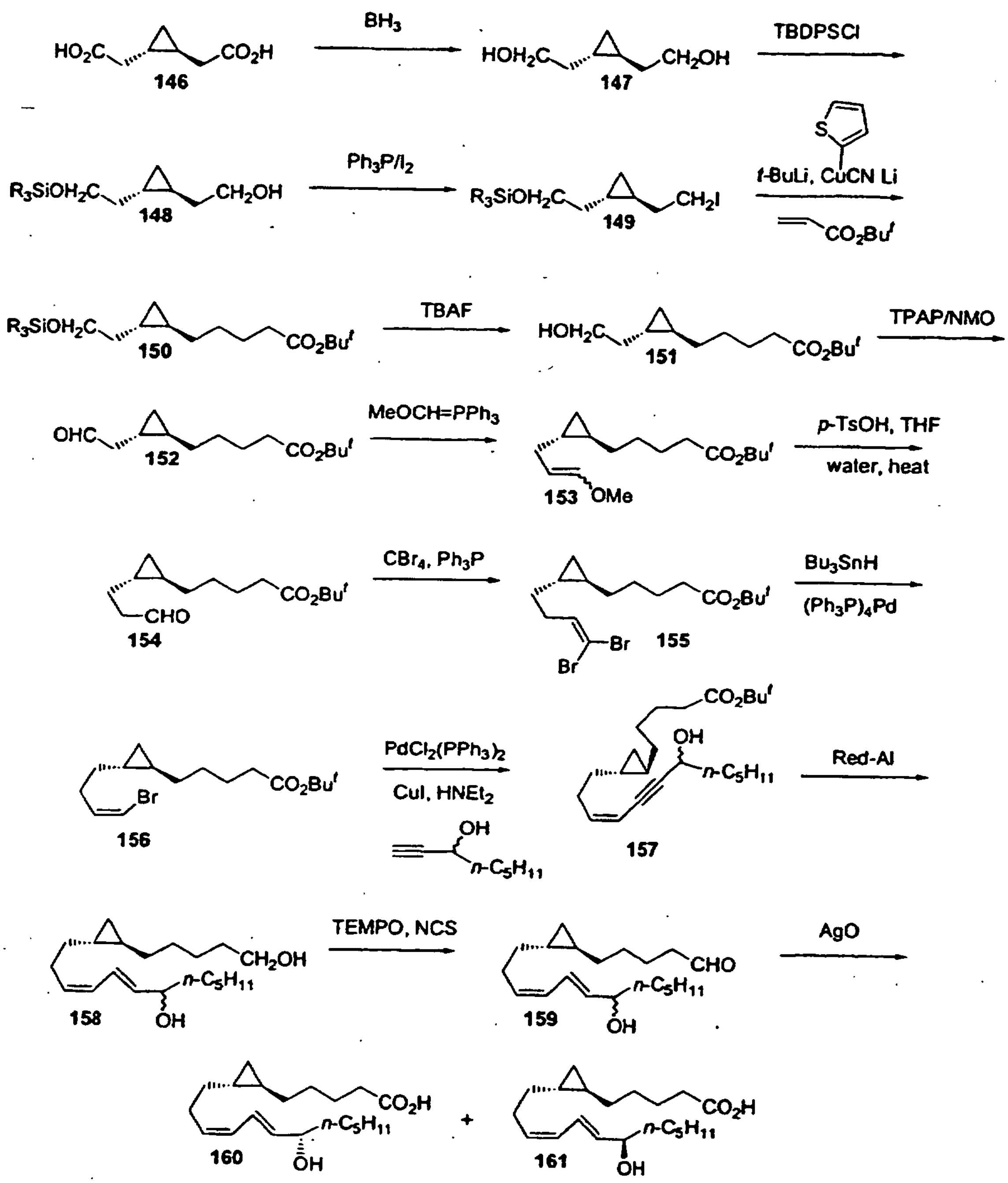


Compounds **160** and **161**

Reduction of diacid **146** (Neset *et. al.*, *Tetrahedron* **1997**, *53*,10459) with $BH_3 \bullet SMe_2$ affords diol **147**, which is silylated with TBDPSCl in the presence of DMAP and imidazole to afford silyl ether **148.** Treatment of **148** with $I_2$ and $PPh_3$ in toluene in the presence of imidazole affords iodide **149. 149** is treated sequentially with with *t*-butyllithium at -78 °C, then lithium (2-thienyl)cyanocuprate, then *t*-butyl acrylate to afford the Michael adduct **150** after quenching

with aqueous acid. **150** is desilylated to alcohol **151** using TBAF in THF. Oxidation of **151** with catalytic TPAP and stoichiometric NMO provides aldehyde **152,** which is converted to enol ether **153** by Wittig reaction with $MeOCH=PPh_3$. Hydrolysis of **153** using TsOH in THF/water with heating affords homologated aldehyde **154,** which is converted to dibromoolefin **155** by condensation with $CBr_4$ in the presence of $PPh_3$. Selective monoreduction of **155** with $Bu_3SnH$ in the presence of catalytic $Pd(PPh_3)_4$ gives *Z*-bromoalkene **156,** which upon treatment with 1-octyn-3-ol, CuI, and catalytic $PdCl_2(PPh_3)_2$ in $HNEt_2$ yields enynol **157. 157** is reduced with Red-Al® in toluene to provide diene diol **158,** which is selectively oxidized to hydroxyaldehyde **159** using TEMPO and stoichiometric NCS. Oxidation of **159** with silver (II) oxide, followed by chromatographic separation of the allyl alcohol diastereomers, affords the α isomer **160** and the β isomer **161**.

**Example 18:**

**Synthesis of 162 and 163**

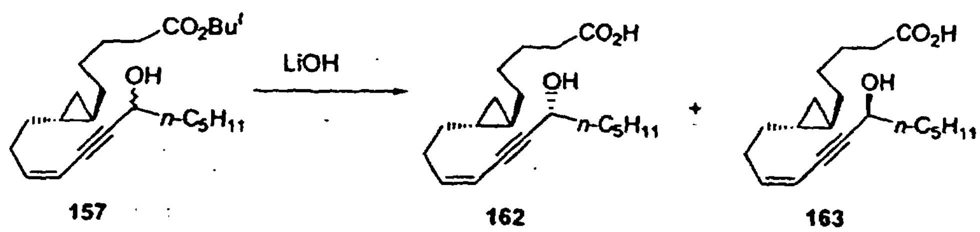


157 162 + 163

Compounds **162** and **163**

Ester **157** is treated with KOH in MeOH/water followed by chromatographic separation of the propargyl alcohol diastereomers to afford targets **162** and **163.**

**Example 19:**

**Synthesis of 168 and 169**

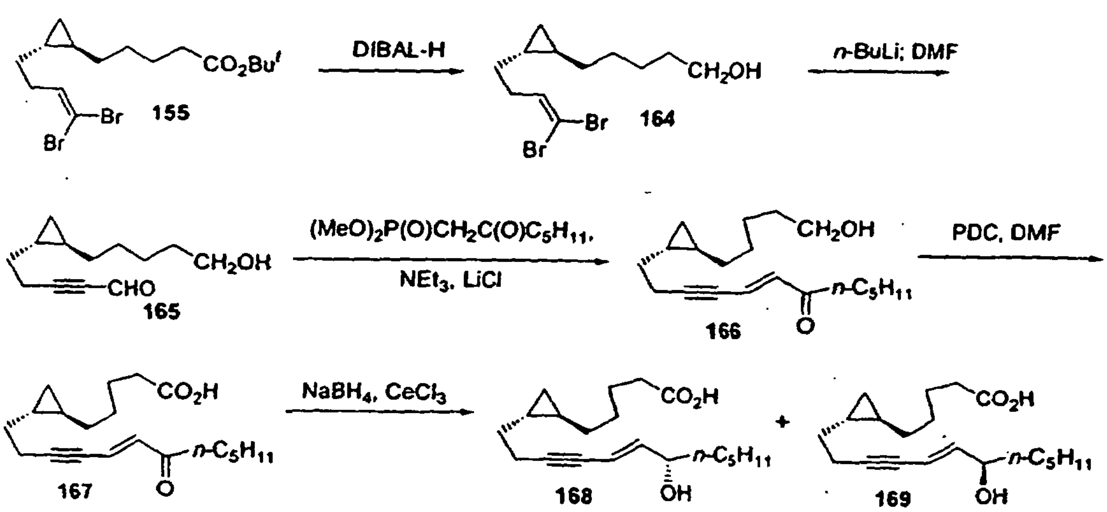


155 164 165 166 167 168 + 169

Compounds **168** and **169**

Reduction of ester **155** with diisobutylaluminum hydride (DIBAL-H) affords alcohol **164,** which is treated with three equivalents of *n*-butyllithium at -78 °C and then with *N*,*N*-dimethylformamide (DMF) to provide ynal **165**. Homer-Emmons condensation of **165** with dimethyl (2-oxoheptyl)phosphonate in the presence of LiCl and $NEt_3$ gives ynenone **166,** which is oxidized to acid **167** using pyridinium dichromate (PDC) in DMF. Reduction of **167** using $NaBH_4$ in the presence of $CeCl_3$, followed by chromatographic separation of the two allyl alcohol diastereomers, affords compounds **168** and **169.**

The compounds of the present invention may be contained in various types of pharmaceutical compositions, in accordance with formulation techniques known to those skilled in the art. Preferably, these compounds will be formulated in solutions for topical ophthalmic administration. The level of peroxy compounds in the HETE derivative raw materials that are used to prepare the pharmaceutical formulations of the present invention may have an impact on the HETE derivative's biological activity. Although the precise relationship has not been defined, it is preferable to use HETE derivative raw material supplies containing peroxy compounds at levels no greater than about 0.3 ppm. Methods for determining peroxy levels are known in the art (e.g., European Pharmacopoeia 1997 3rd Ed., Method 2.5.5 - Peroxide Value).

The ophthalmic compositions of the present invention will include one or more compounds of the present invention in a pharmaceutically acceptable vehicle. Various types of vehicles may be used. Aqueous solutions are generally preferred, based on ease of formulation, biological compatibility, as well as a patient's ability to easily administer such compositions by means of instilling one to two drops of the solutions in the affected eyes. However, the compounds of the present invention may also be readily incorporated into other types of compositions, such as suspensions, viscous or semi-viscous gels, or other types of solid or semi-solid compositions. Suspensions may be preferred for those compounds of the present invention which are less soluble in water. The ophthalmic compositions of the present invention may also include various other ingredients, such as buffers, preservatives, co-solvents and viscosity building agents.

An appropriate buffer system (e.g., sodium phosphate, sodium acetate or sodium borate) may be added to prevent pH drift under storage conditions.

Antioxidants may be added to compositions of the present invention to protect the active ingredients from oxidation during storage. Examples of such antioxidants include vitamin E and analogs thereof, ascorbic acid and butylated hydroxytoluene (BHT).

Ophthalmic products are typically packaged in multidose form. Preservatives are thus required to prevent microbial contamination during use. Suitable preservatives include: benzalkonium chloride, thimerosal, chlorobutanol, methyl paraben, propyl paraben, phenylethyl alcohol, edetate disodium, sorbic acid, polyquaternium-1, or other agents known to those skilled in the art. Such preservatives are typically employed at a level of from 0.001 to 1.0% weight/volume ("% w/v").

In general, the doses used for the above described purposes will vary, but will be in an effective amount to increase mucin production in the eye and thus eliminate or improve dry eye conditions. As used herein, the term "pharmaceutically effective amount" refers to an amount which improves the dry eye condition in a human patient. When the compositions are dosed topically, they will generally be in a concentration range of from 0.001 to about 1.0% w/v, with 1-2 drops administered 1-4 times per day.

As used herein, the term "pharmaceutically acceptable carrier" refers to any vehicle which, when formulated, is safe, and provides the appropriate delivery for the desired route of administration of an effective amount of at least one compound of the present invention.

In one embodiment, the ophthalmic compositions of the present invention will contain ethanol in addition to a compound of formula (I). As used herein, "an effective concentration of ethanol" refers to a concentration that enhances the biological efficacy of the formula (I) compositions *in vivo.* In general, the concentration of ethanol necessary for the enhancement of the compounds of formula (I) is believed to be somewhat proportional to the concentration of the formula (I) compound(s) administered. If a relatively high concentration of formula (I) compound(s), e.g., above 0.01% w/v, is administered, the concentration of ethanol in such compositions may be proportionally less than analogous compositions containing lower concentrations of formula (I) compounds. In general, however, the ethanol concentration contained in the ophthalmic compositions of the present invention will range from about 0.001-2% w/v. Compositions containing formula (I) concentrations of about 0.00001-0.02% w/v preferably will contain ethanol in a concentration of about 0.005-0.2% w/v, and most preferably, about 0.02-0.10% w/v. An example of a topically administrable ophthalmic formulation according to this embodiment of the present invention is provided below.

## Example 20

| Ingredient | Amount (% w/v) |
|---|---|
| Compound of formula (I) | 0.00001-0.01 |
| Ethanol | 0.0505 |
| Polyoxyl 40 Stearate | 0.1 |
| Boric Acid | 0.25 |
| Sodium Chloride | 0.75 |
| Disodium Edetate | 0.01 |
| Polyquaternium-1 | 0.001 |
| NaOH/HCl | q.s., pH = 7.5 |
| Purified Water | q.s. 100% |

The above composition is prepared by the following method. The batch quantities of polyoxyl 40 stearate, boric acid, sodium chloride, disodium edetate, and polyquaternium-1 are weighed and dissolved by stirring in 90% of the batch quantity of purified water. The pH is adjusted to $7.5 \pm 0.1$ with NaOH and/or HCl. Under yellow light or reduced lighting, the batch quantity of a compound of formula (I) as a stock solution in ethanol and the additional quantity of ethanol necessary for the batch are measured and added. Purified water is added to q.s. to 100%. The mixture is stirred for five minutes to homogenize and then filtered through a sterilizing filter membrane into a sterile recipient.

Preferably, the above process is performed using glass, plastic or other non-metallic containers or containers lined with such materials.

## Claims

1. A composition for the treatment of dry eye and other disorders requiring the wetting of the eye comprising a pharmaceutically acceptable carrier and a pharmaceutically effective amount of one or more compounds of the following

$$n\text{-}C_5H_{11}\text{-}Y\text{-}A\text{-}CH_2\text{-}R^1$$

**I** I

formula **I**:
wherein:

$R^1$ is $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$, or 2,3,4,5-tetrazol-1-yl, where:

R is H or a pharmaceutically acceptable cation, or $CO_2R$ forms a pharmaceutically acceptable ester moiety;
$NR^2R^3$, $NR^5R^6$ are the same or different and comprise a free or functionally modified amino group;
$OR^4$ comprises a free or functionally modified hydroxy group;
Hal is F, Cl, Br, or I;
$R^{20}$ is H, alkyl, acyl;
$R^{21}$ is H or a pharmaceutically acceptable cation, or $COSR^{21}$ forms a pharmaceutically acceptable

thioester moiety;

A is $L_1$-$A_2$-$L_2$, $L_3$-$A_2$-$L_4$, or $L_5$-$A_2$-$L_3$.

$A_2$ is

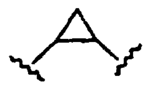 ;

$L_1$ is $CH_2$-B-D;

B and D are the same or different and are $CH_2CH_2$, CH=CH, or C=C;

$L_2$ is $CH_2$-K-$CH_2CH_2$;

K is $CH_2CH_2$, CH=CH, or C≡C;

$L_3$ is $CH_2CH_2CH_2$, $CH_2$CH=CH, $CH_2$C≡C, CH=$CHCH_2$, C≡$CCH_2$, or CH=C=CH;

$L_4$ is X-$CH_2CH_2$;

X is $CH_2CH_2$CH=CH, $CH_2CH_2$C≡C, $CH_2CH_2CH_2CH_2$, $CH_2$CH=$CHCH_2$,

$CH_2$C≡$CCH_2$, CH=$CHCH_2CH_2$, C≡$CCH_2CH_2$, $CH_2$CH=C=CH, or CH=C=$CHCH_2$;

$L_5$ is $CH_2CH_2$-B-D; and

Y is C(O) (*i.e.* a carbonyl group) or Y is

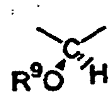 or 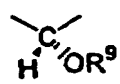 ;

wherein $R^9$O constitutes a free or functionally modified hydroxy group.

**2.** The composition of Claim 1, wherein for the compound of formula I:

$R^1$ is $CO_2R$, where R is H or an ophthalmically acceptable cationic salt, or $CO_2R$ forms an ophthalmically acceptable ester moiety;

A is $L_1$-$A_2$-$L_2$;

B is C≡C or *cis*-CH=CH and D is C=C or *trans*-CH=CH;

K is *cis*-CH=CH; and

Y is

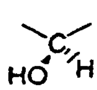 or 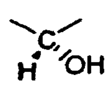

**3.** The composition of Claim 2, wherein the compound is selected from the group consisting of:

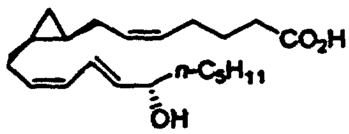 ; 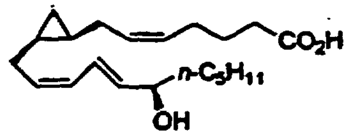 ; 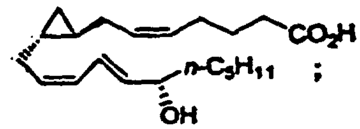 ;

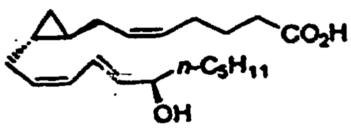 ; 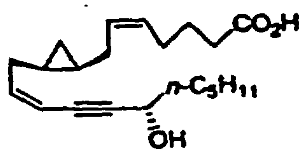 ; 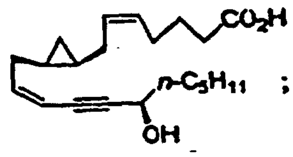 ;

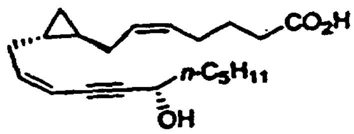 ; 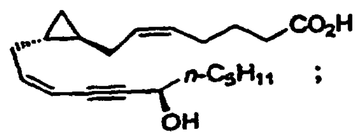 ; 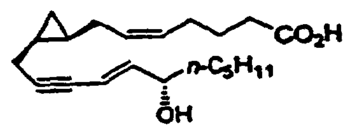

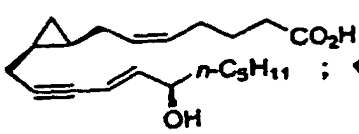 ; 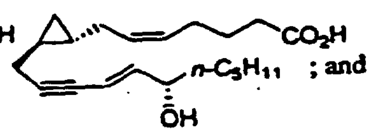 ; and 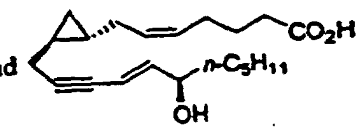 .

4. The composition of Claim 1, wherein for the compound of formula **I**:

$R^1$ is $CO_2R$, where R is H or an ophthalmically acceptable cationic salt, or $CO_2R$ forms an ophthalmically acceptable ester moiety;

A is $L_3$-$A_2$-$L_4$;

$L_3$ is *trans*-$CH_2CH{=}CH$, *trans*-$CH{=}CHCH_2$, or $CH_2C{\equiv}C$;

X is trans-$CH_2CH_2CH{=}CH$, $CH_2CH_2C{\equiv}C$, trans-$CH_2CH{=}CHCH_2$, or trans-$CH{=}CHCH_2CH_2$; and

Y is

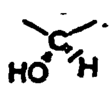 or 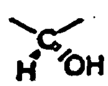 .

5. The composition of Claim 1, wherein the compound of formula **I** is selected from the group consisting of:

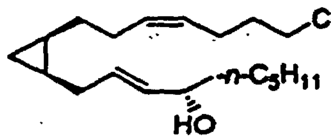 ; 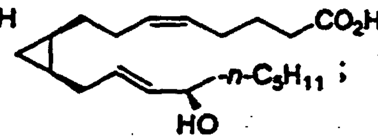 ; 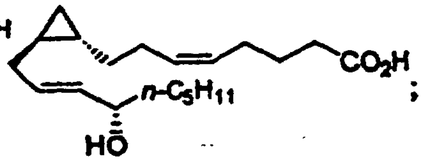 ;

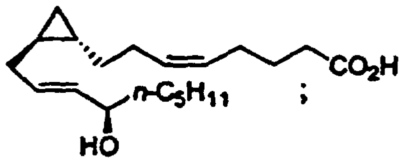 ; 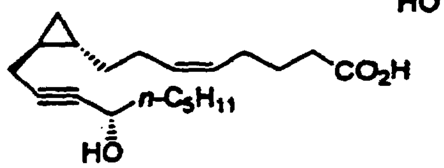 ; and

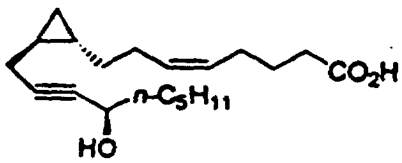 .

6. The composition of Claim 1, wherein for the compound of formula **I**:

$R^1$ is $CO_2R$, where R is H or an ophthalmically acceptable cationic salt, or $CO_2R$ forms an ophthalmically acceptable ester moiety;

A is $L_5$-$A_2$-$L_3$;

$L_3$ is *trans*-$CH_2CH=CH$, *trans*-$CH=CHCH_2$, $CH_2C\equiv C$, or $CH_2CH_2CH_2$;

B is *cis*-CH=CH or C≡C;

D is *trans*-CH=CH or C≡C; and

Y is

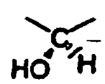 or 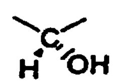 .

7. The composition of Claim 6, wherein the compound is selected from the group consisting of:

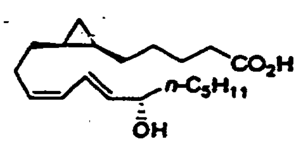 ; 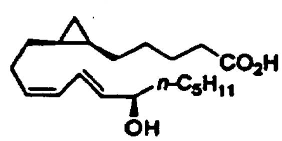 ; 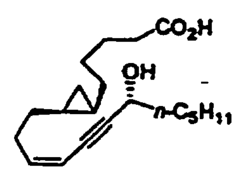 ;

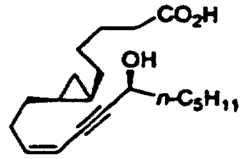 ; 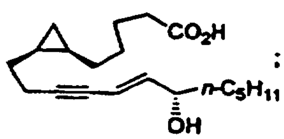 ; 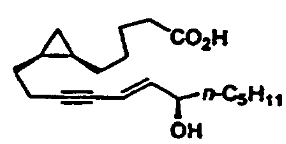 ;

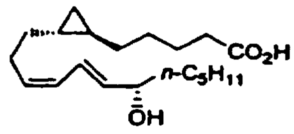 ; 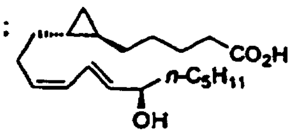 ; 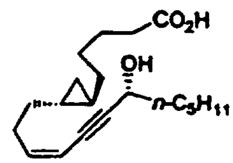 ;

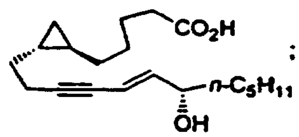 ; and 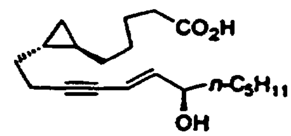

**8.** The composition of Claim 1, wherein the composition is suitable for topical administration to the eye.

**9.** Use of one or more compounds according to formula I:

$$n\text{-}C_5H_{11}\text{-}Y\text{-}A\text{-}CH_2\text{-}R^1 \qquad \text{I}$$

wherein:

$R^1$ is $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$, or 2,3,4,5-tetrazol-1-yl, where:

R is H or a pharmaceutically acceptable cation, or $CO_2R$ forms a pharmaceutically acceptable ester moiety;
$NR^2R^3$, $NR^5R^6$ are the same or different and comprise a free or functionally modified amino group;
$OR^4$ comprises a free or functionally modified hydroxy group;
Hal is F, Cl, Br, or I;
$R^{20}$ is H, alkyl, acyl;
$R^{21}$ is H or a pharmaceutically acceptable cation, or $COSR^{21}$ forms a pharmaceutically acceptable thioester moiety;

A is $L_1$-$A_2$-$L_2$, $L_3$-$A_2$-$L_4$, or $L_5$-$A_2$-$L_3$;

$A_2$ is

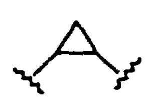 ;

$L_1$ is $CH_2$-B-D;

B and D are the same or different and are $CH_2CH_2$, CH=CH, or C≡C;

$L_2$ is $CH_2$-K-$CH_2CH_2$;

K is $CH_2CH_2$, CH=CH, or C≡C;

$L_3$ is $CH_2CH_2CH_2$, $CH_2CH$=CH, $CH_2C$≡C, CH=$CHCH_2$, C≡$CCH_2$, or CH=C=CH;

$L_4$ is X-$CH_2CH_2$;

X is $CH_2CH_2CH{=}CH$, $CH_2CH_2C{\equiv}C$, $CH_2CH_2CH_2CH_2$, $CH_2CH{=}CHCH_2$, $CH_2C{\equiv}CCH_2$, $CH{=}CHCH_2CH_2$, $C{\equiv}CCH_2CH_2$, $CH_2CH{=}C{=}CH$, or $CH{=}C{=}CHCH_2$;

$L_5$ is $CH_2CH_2$-B-D; and

Y is C(O) (*i.e.* a carbonyl group) or Y is

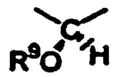

or

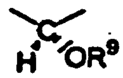

;

wherein $R^9O$ constitutes a free or functionally modified hydroxy group for the preparation of a pharmaceutical composition for the treatment of dry eye and other disorders requiring the wetting of the eye in mammals.

**10.** The use of Claim 9, wherein the mammal is a human and the compound is administered topically.

**11.** The use of Claim 9, wherein for the compound of formula **I**:

$R^1$ is $CO_2R$, where R is H or an ophthalmically acceptable cationic salt, or $CO_2R$ forms an ophthalmically acceptable ester moiety;
A is $L_1$-$A_2$-$L_2$;
B is $C{\equiv}C$ or *cis*-CH=CH and D is $C{\equiv}C$ or *trans*-CH=CH;
K is *cis*-CH=CH; and
Y is

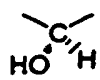

or

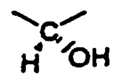

**12.** The use of Claim 11, wherein the compound is selected from the group consisting of:

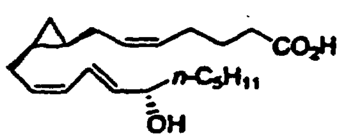

;

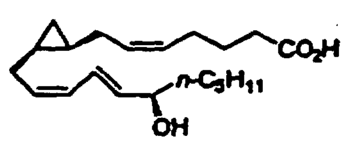

;

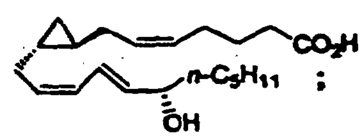

;

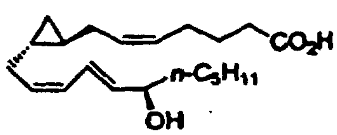

;

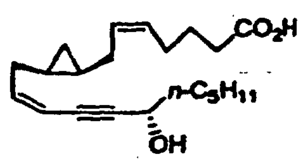

;

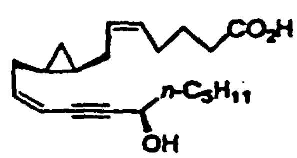

;

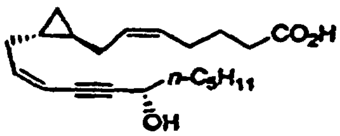 ; 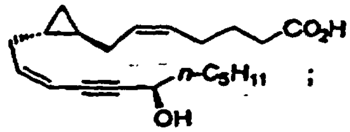 ; 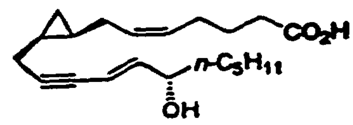 ;

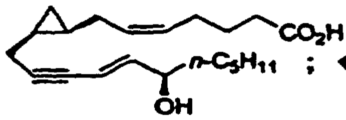 ; 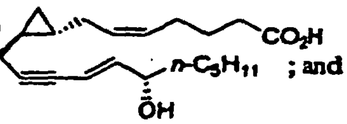 ; and 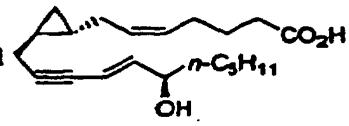 .

**13.** The use of Claim 9, wherein for the compound of formula I:

$R^1$ is $CO_2R$, where R is H or an ophthalmically acceptable cationic salt, or $CO_2R$ forms an ophthalmically acceptable ester moiety;

A is $L_3$-$A_2$-$L_4$;

$L_3$ is *trans*-$CH_2CH{=}CH$, *trans*-$CH{=}CHCH_2$, or $CH_2C{\equiv}C$;

X is trans-$CH_2CH_2CH{=}CH$, $CH_2CH_2C{\equiv}C$, trans-$CH_2CH{=}CHCH_2$, or trans-$CH{=}CHCH_2CH_2$; and

Y is

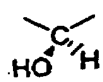 or 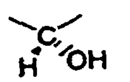 .

**14.** The use of Claim 9, wherein the compound is selected from the group consisting of:

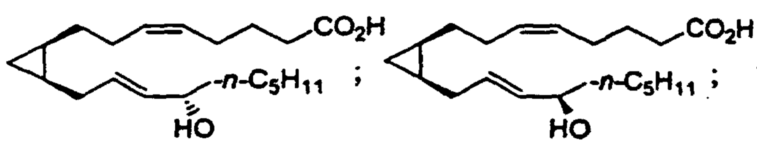 ; 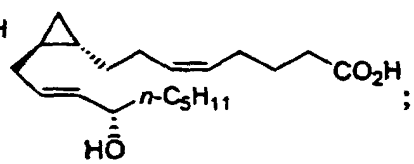 ;

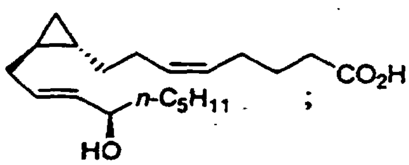 ; 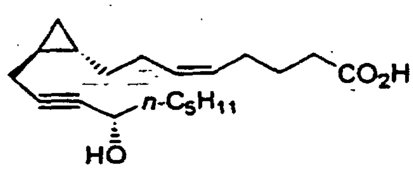 ; and

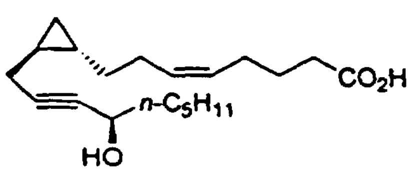 .

**15.** The use of Claim 9, wherein for the compound of formula **I**:

$R^1$ is $CO_2R$, where R is H or an ophthalmically acceptable cationic salt, or $CO_2R$ forms an ophthalmically acceptable ester moiety;

A is $L_5$-$A_2$-$L_3$;

$L_3$ is *trans*-$CH_2CH$=CH, *trans*-CH=$CHCH_2$, $CH_2C{\equiv}C$, or $CH_2CH_2CH_2$;

B is *cis*-CH=CH or C≡C;

D is *trans*-CH=CH or C≡C; and

Y is

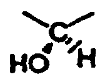

or

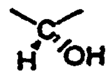

.

**16.** The use of Claim 15, wherein the compound is selected from the group consisting of:

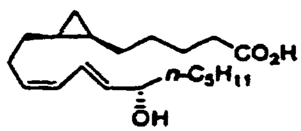
;
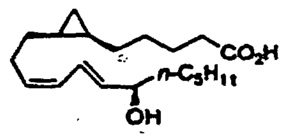
;
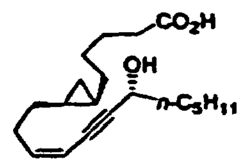
;
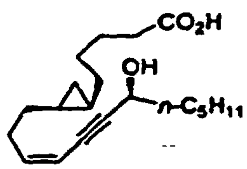
;
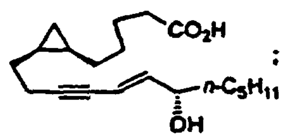
;
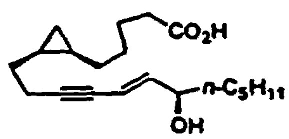
;
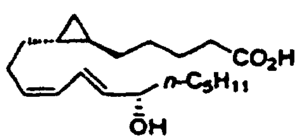
;
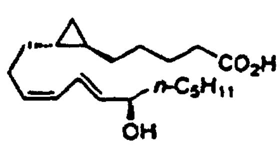
;
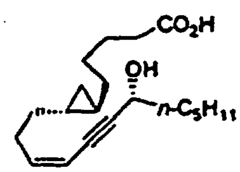
;
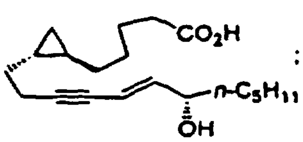
; and
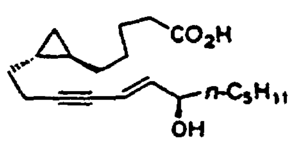
.

**17.** The use of Claim 9 wherein the dry eye and other disorders requiring the wetting of the eye is symptoms of dry eye associated with refractive surgery.

**18.** A compound of formula **I**:

$$n\text{-}C_5H_{11}\text{-}Y\text{-}A\text{-}CH_2\text{-}R^1$$

**I** I

wherein:

$R^1$ is $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$, or 2,3,4,5-tetrazol-1-yl, where:

R is H or a pharmaceutically acceptable cation, or $CO_2R$ forms a pharmaceutically acceptable ester moiety;
$NR^2R^3$, $NR^5R^6$ are the same or different and comprise a free or functionally modified amino group;
$OR^4$ comprises a free or functionally modified hydroxy group;
Hal is F, Cl, Br, or I;
$R^{20}$ is H, alkyl, acyl;

$R^{21}$ is H or a pharmaceutically acceptable cation, or $COSR^{21}$ forms a pharmaceutically acceptable thioester moiety; A is $L_1$-$A_2$-$L_2$, $L_3$-$A_2$-$L_4$, or $L_5$-$A_2$-$L_3$;

$A_2$ is

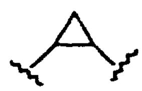 ;

$L_1$ is $CH_2$-B-D;

B and D are the same or different and are $CH_2CH_2$, CH=CH, or C≡C;

$L_2$ is $CH_2$-K-$CH_2CH_2$;

K is $CH_2CH_2$, CH=CH, or C≡C;

$L_3$ is $CH_2CH_2CH_2$, $CH_2CH$=CH, $CH_2C$≡C, CH=$CHCH_2$, C≡$CCH_2$, or CH=C=CH;

$L_4$ is X-$CH_2CH_2$;

X is $CH_2CH_2CH$=CH, $CH_2CH_2C$≡C, $CH_2CH_2CH_2CH_2$, $CH_2CH$=$CHCH_2$,

$CH_2C$≡$CCH_2$, CH=$CHCH_2CH_2$, C≡$CCH_2CH_2$, $CH_2CH$=C=CH, or CH=C=$CHCH_2$;

$L_5$ is $CH_2CH_2$-B-D; and

Y is C(O) (*i.e.* a carbonyl group) or Y is

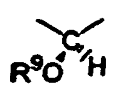
or
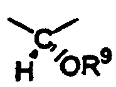 ;

wherein $R^9O$ constitutes a free or functionally modified hydroxy group.

**19.** The compound of Claim 18, wherein:

$R^1$ is $CO_2R$, where R is H or an ophthalmically acceptable cationic salt, or $CO_2R$ forms an ophthalmically

acceptable ester moiety;

A is $L_1$-$A_2$-$L_2$;

B is C≡C or *cis*-CH=CH and D is C≡C or *trans*-CH=CH;

K is *cis*-CH=CH; and

Y is

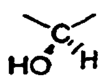
or
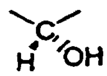
.

**20.** The compound of Claim 19, wherein the compound is selected from the group consisting of:

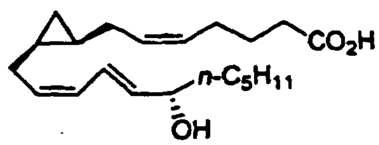
;
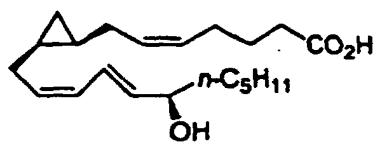
;

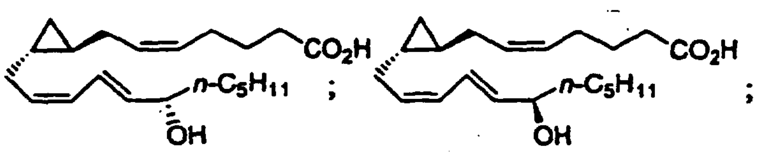
; ;

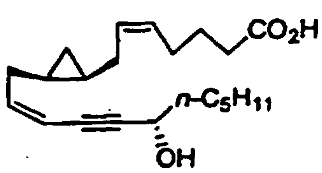
;
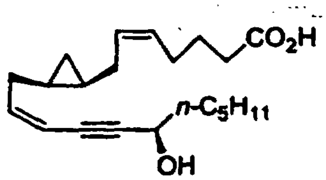
;
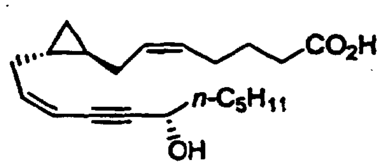
;

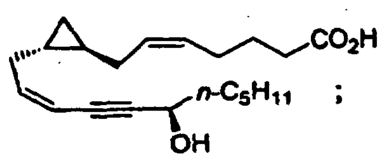
;
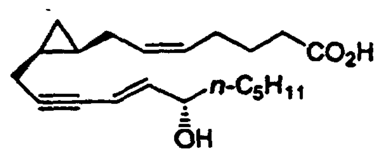
;

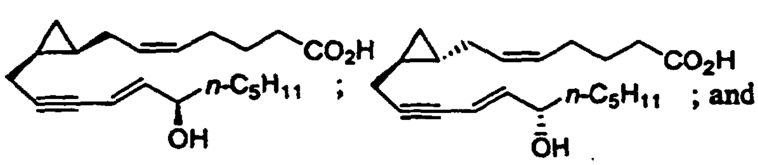
; ; and
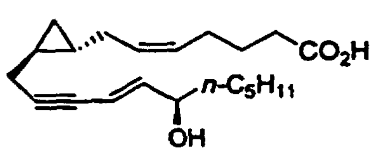
.

**21.** The compound of Claim 18, wherein:

$R^1$ is $CO_2R$, where R is H or an ophthalmically acceptable cationic salt, or $CO_2R$ forms an ophthalmically

acceptable ester moiety;
A is $L_3$-$A_2$-$L_4$;
$L_3$ is *trans*-$CH_2CH$=CH, *trans*-CH=$CHCH_2$, or $CH_2C{\equiv}C$;
X is trans-$CH_2CH_2CH$=CH, $CH_2CH_2C{\equiv}C$, trans-$CH_2CH$=$CHCH_2$, or trans-CH=$CHCH_2CH_2$; and
Y is

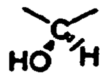
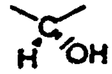

or

**22.** The compound of Claim 21, wherein the compound is selected from the group consisting of:

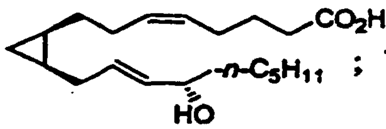 ; 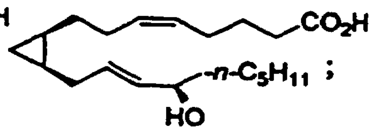 ; 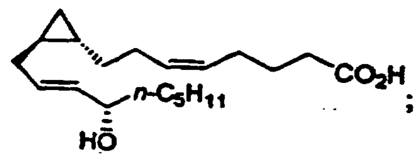 ;

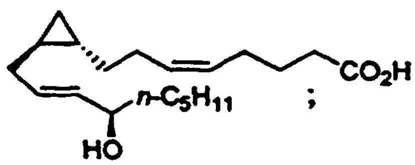 ; 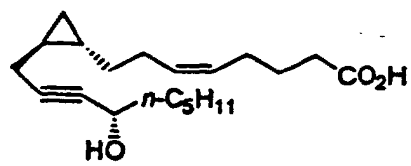 ;

and

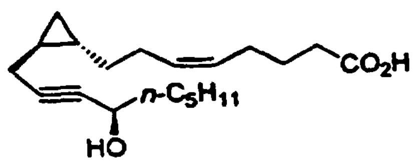

**23.** The compound of claim 18, wherein:

$R^1$ is $CO_2R$, where R is H or an ophthalmically acceptable cationic salt, or $CO_2R$ forms an ophthalmically acceptable ester moiety;
A is $L_5$-$A_2$-$L_3$;
$L_3$ is *trans*-$CH_2CH$=CH, *trans*-CH=$CHCH_2$, $CH_2C{\equiv}C$, or $CH_2CH_2CH_2$;
B is *cis*-CH=CH or C≡C;
D is *trans*-CH=CH or C≡C; and
Y is

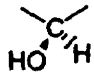
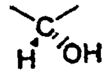

or .

**24.** The compound of Claim 23, wherein the compound is selected from the group consisting of:

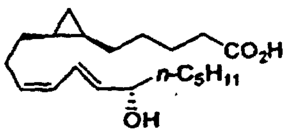 ; 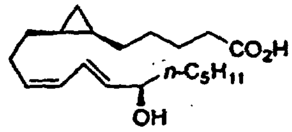 ; 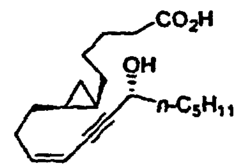 ;

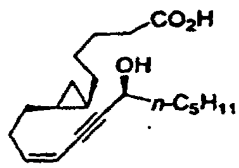 ; 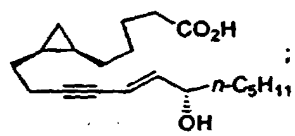 ; 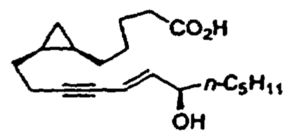 ;

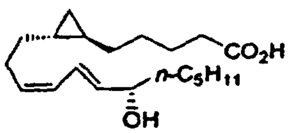 ; 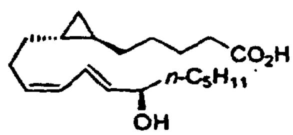 ; 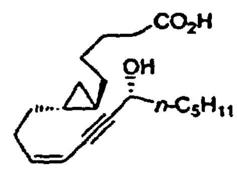 ;

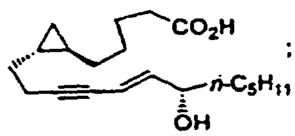 ; and 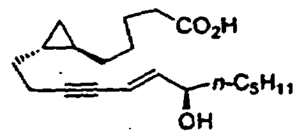 .

## Patentansprüche

1. Zusammensetzung zur Behandlung von trockenem Auge und anderen Leiden, die das Benetzen des Auges erfordern, enthaltend einen pharmazeutisch annehmbaren Träger und eine pharmazeutisch wirksame Menge einer oder mehrerer Verbindungen der folgenden Formel I

$$n\text{-}C_5H_{11}\text{-}Y\text{-}A\text{-}CH_2\text{-}R^1 \qquad \text{I}$$

worin

$R^1$ $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$ oder 2,3,4,5-Tetrazol-1-yl ist, wobei

R H oder ein pharmazeutisch annehmbares Kation ist oder $CO_2R$ einen pharmazeutisch annehmbaren Esteranteil bildet;
$NR^2R^3$, $NR^5R^6$ gleich oder verschieden sind und eine freie oder funktionell modifizierte Aminogruppe beinhalten;
$OR^4$ eine freie oder funktionell modifizierte Hydroxygruppe beinhaltet;
Hal F, Cl, Br oder I ist;
$R^{20}$ H, Alkyl, Acyl ist;
$R^{21}$ H oder ein pharmazeutisch annehmbares Kation ist oder $COSR^{21}$ einen pharmazeutisch annehmbaren Thioesteranteil bildet;

A $L_1$-$A_2$-$L_2$, $L_3$-$A_2$-$L_4$ oder $L_5$-$A_2$-$L_3$ ist,
$A_2$

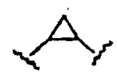

ist;
$L_1$ $CH_2$-B-D ist;
B und D gleich oder verschieden sind und $CH_2CH_2$, CH=CH oder C≡C bedeuten;
$L_2$ $CH_2$-K-$CH_2CH_2$ ist;
K $CH_2CH_2$, CH=CH oder C≡C ist;
$L_3$ $CH_2CH_2CH_2$, $CH_2CH$=CH, $CH_2C$≡C, CH=$CHCH_2$, C≡$CCH_2$ oder CH=C=CH ist;
$L_4$ X-$CH_2CH_2$ ist;
X $CH_2CH_2CH$=CH, $CH_2CH_2C$≡C, $CH_2CH_2CH_2CH_2$, $CH_2CH$=$CHCH_2$, $CH_2C$≡$CCH_2$, CH=$CHCH_2CH_2$, C≡$CCH_2CH_2$, $CH_2CH$=C=CH oder CH=C=$CHCH_2$ ist;
$L_5$ $CH_2CH_2$-B-D ist und
Y C(O) (d.h. eine Carbonylgruppe) ist oder Y

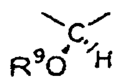

oder

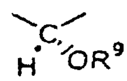

ist, wobei $R^9O$ eine freie oder funktionell modifizierte Hydroxygruppe bildet.

**2.** Zusammensetzung nach Anspruch 1, wobei für die Verbindung der Formel I

$R^1$ $CO_2R$ ist, wobei R H oder ein ophthalmisch annehmbares kationisches Salz ist oder $CO_2R$ einen ophthalmisch annehmbaren Esteranteil bildet;
A $L_1$-$A_2$-$L_2$ ist;
B C≡C oder *cis*-CH=CH ist und D C≡C oder *trans*-CH=CH ist;
K *cis*-CH=CH ist und
Y

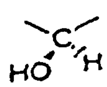

oder

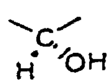

ist.

**3.** Zusammensetzung nach Anspruch 2, wobei die Verbindung ausgewählt ist aus der Gruppe bestehend aus

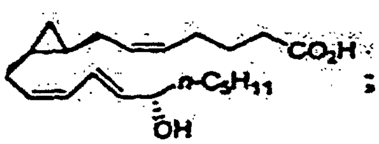

;

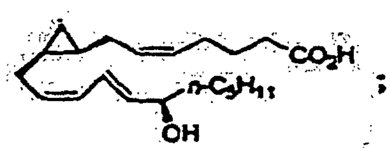

;

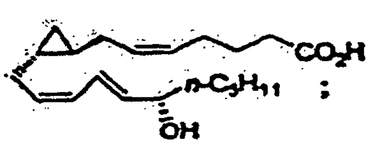

;

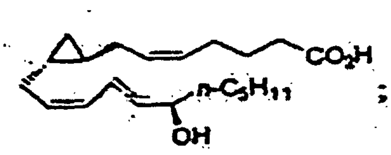 ; 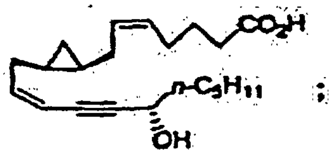 ; 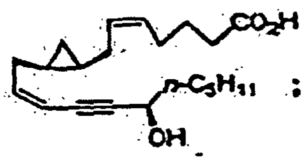 ;

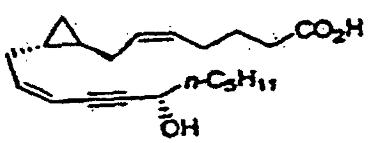 ; 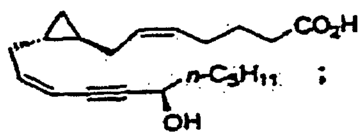 ; 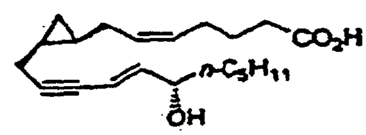

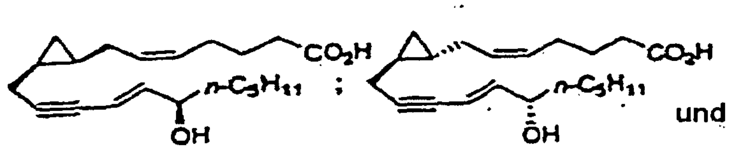 ; und 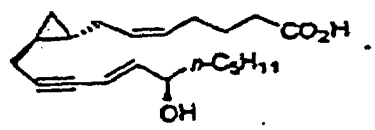 .

4. Zusammensetzung nach Anspruch 1, wobei in der Verbindung der Formel I

$R^1$ $CO_2R$ ist, wobei R H oder ein ophthalmisch annehmbares kationisches Salz ist, oder $CO_2R$ einen ophthalmisch annehmbaren Esteranteil bildet;
A $L_3$-$A_2$-$L_4$ ist;
$L_3$ *trans*-$CH_2CH$=CH, *trans*-CH=$CHCH_2$ oder $CH_2C{\equiv}C$ ist;
X *trans*-$CH_2CH_2CH$=CH, $CH_2CH_2C{\equiv}C$, *trans*-$CH_2CH$=$CHCH_2$ oder *trans*-CH=$CHCH_2CH_2$ ist und
Y

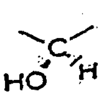 oder 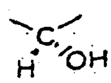

ist.

5. Zusammensetzung nach Anspruch 1, wobei die Verbindung der Formel I ausgewählt ist aus der Gruppe bestehend aus

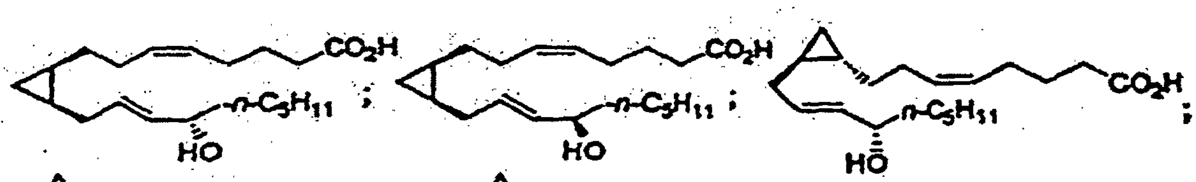 ; ; ;

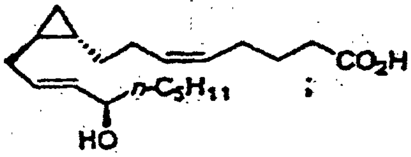 ; 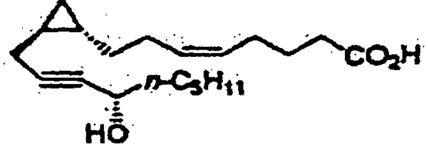 und

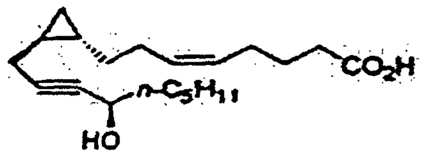 .

**6.** Zusammensetzung nach Anspruch 1, wobei für die Verbindung der Formel I

$R^1$ $CO_2R$ ist, wobei R H oder ein ophthalmisch annehmbares kationisches Salz ist, oder $CO_2R$ einen ophthalmisch annehmbaren Esteranteil bildet;
A $L_5$-$A_2$-$L_3$ ist;
$L_3$ *trans*-$CH_2CH{=}CH$, *trans*-$CH{=}CHCH_2$, $CH_2C{\equiv}C$ oder $CH_2CH_2CH_2$ ist;
B *cis*-$CH{=}CH$ oder $C{\equiv}C$ ist;
D *trans*-$CH{=}CH$ oder $C{\equiv}C$ ist und
Y

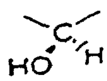 oder 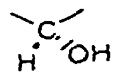

ist.

**7.** Zusammensetzung nach Anspruch 6, wobei die Verbindung ausgewählt ist aus der Gruppe bestehend aus

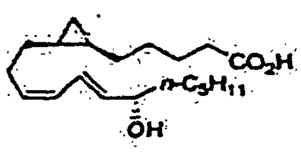 ; 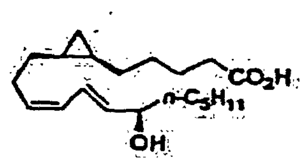 ; 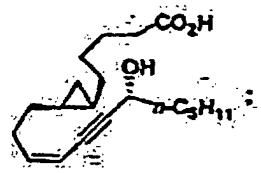 ;

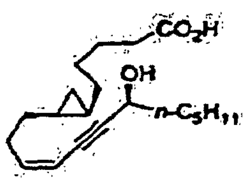 ; 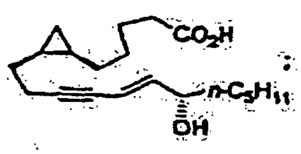 ; 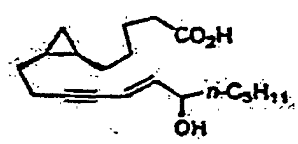

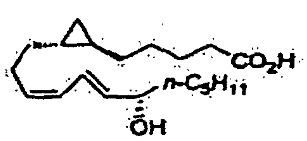 ; 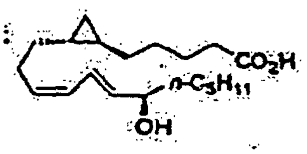 ; 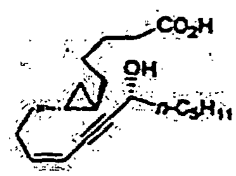 ;

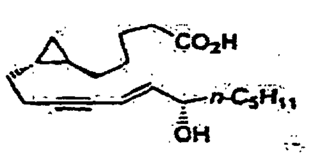 und 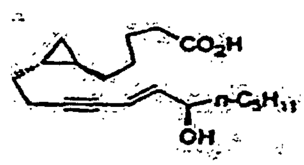

**8.** Zusammensetzung nach Anspruch 1, wobei die Zusammensetzung für die topische Anwendung am Auge geeignet ist.

**9.** Verwendung einer oder mehrerer Verbindungen der Formel I

$$n\text{-}C_5H_{11}\text{-}Y\text{-}A\text{-}CH_2\text{-}R^1 \qquad \text{I}$$

worin

$R^1$ $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$ oder 2,3,4,5-Tetrazol-1-yl ist, wobei

R H oder ein pharmazeutisch annehmbares Kation ist oder $CO_2R$ einen pharmazeutisch annehmbaren Esteranteil bildet;
$NR^2R^3$, $NR^5R^6$ gleich oder verschieden sind und eine freie oder funktionell modifizierte Aminogruppe beinhalten;
$OR^4$ eine freie oder funktionell modifizierte Hydroxygruppe beinhaltet;
Hal F, Cl, Br oder I ist;
$R^{20}$ H, Alkyl, Acyl ist;
$R^{21}$ H oder ein pharmazeutisch annehmbares Kation ist oder $COSR^{21}$ einen pharmazeutisch annehmbaren Thioesteranteil bildet;

A $L_1$-$A_2$-$L_2$, $L_3$-$A_2$-$L_4$ oder $L_5$-$A_2$-$L_3$ ist,
$A_2$

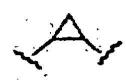

ist;
$L_1$ $CH_2$-B-D ist;
B und D gleich oder verschieden sind und $CH_2CH_2$, CH=CH oder C≡C bedeuten;
$L_2$ $CH_2$-K-$CH_2CH_2$ ist;
K $CH_2CH_2$, CH=CH oder C≡C ist;
$L_3$ $CH_2CH_2CH_2$, $CH_2CH$=CH, $CH_2C$≡C, CH=$CHCH_2$, C≡$CCH_2$ oder CH=C=CH ist;
$L_4$ X-$CH_2CH_2$ ist;
X $CH_2CH_2CH$=CH, $CH_2CH_2C$≡C, $CH_2CH_2CH_2CH_2$, $CH_2CH$=$CHCH_2$, $CH_2C$≡$CCH_2$, CH=$CHCH_2CH_2$, C≡$CCH_2CH_2$, $CH_2CH$=C=CH oder CH=C=$CHCH_2$ ist;
$L_5$ $CH_2CH_2$-B-D ist und
Y C(O) (d.h. eine Carbonylgruppe) ist oder Y

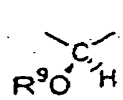

oder

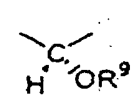

ist, wobei $R^9O$ eine freie oder funktionell modifizierte Hydroxygruppe bildet zur Herstellung einer pharmazeutischen Zusammensetzung zur Behandlung von trockenem Auge und anderen Leiden, die das Benetzen des Auges bei Säugetieren erfordern.

**10.** Verwendung nach Anspruch 9, wobei das Säugetier ein Mensch ist und die Verbindung topisch verabreicht wird.

**11.** Verwendung nach Anspruch 9, wobei in der Verbindung der Formel I

$R^1$ $CO_2R$ ist, wobei R H ist oder ein ophthalmisch annehmbares kationisches Salz ist oder $CO_2R$ einen oph-

thalmisch annehmbaren Esteranteil bildet;
A $L_1$-$A_2$-$L_2$ ist;
B C≡C oder *cis*-CH=CH ist und D C≡C oder *trans*-CH=CH ist;
K *cis*-CH=CH ist und
Y

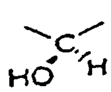 oder 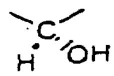

ist.

**12.** Verwendung nach Anspruch 11, wobei die Verbindung ausgewählt ist aus der Gruppe bestehend aus

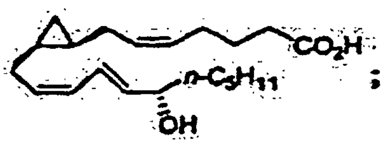 ; 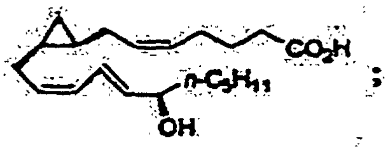 ; 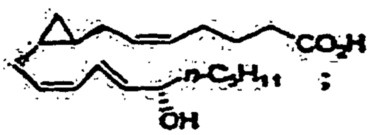 ;

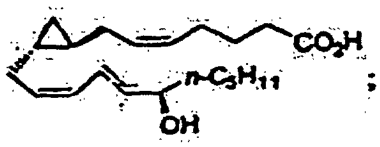 ; 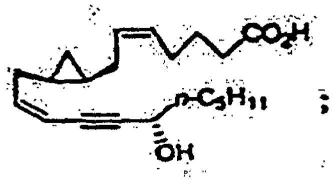 ; 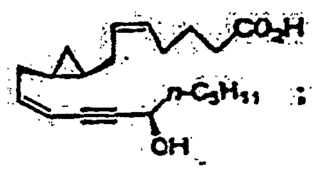 ;

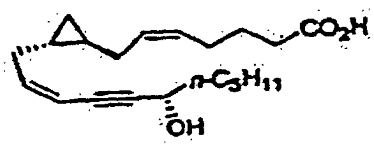 ; 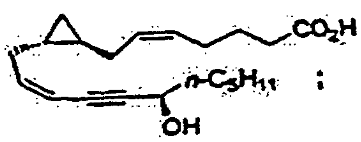 ; 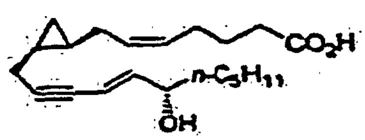

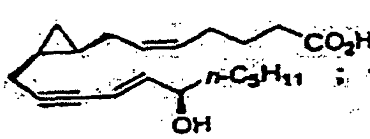 ; 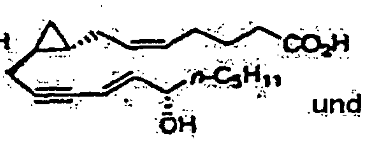 und 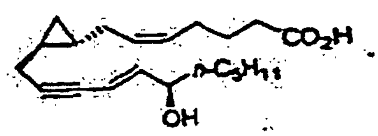

**13.** Verwendung nach Anspruch 9, wobei für die Verbindung der Formel I

$R^1$ $CO_2R$ ist, wobei R H oder ein ophthalmisch annehmbares kationisches Salz ist, oder $CO_2R$ einen ophthalmisch annehmbaren Esteranteil bildet;
A $L_3$-$A_2$-$L_4$ ist;
$L_3$ *trans*-$CH_2CH$=CH, *trans*-CH=$CHCH_2$ oder $CH_2C$≡C ist;
X *trans*-$CH_2CH_2CH$=CH, $CH_2CH_2C$≡C, *trans*-$CH_2CH$=$CHCH_2$ oder *trans*-CH=$CHCH_2CH_2$ ist und
Y

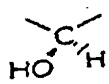 oder 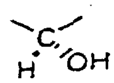

ist.

**14.** Verwendung nach Anspruch 9, wobei die Verbindung ausgewählt ist aus der Gruppe bestehend aus

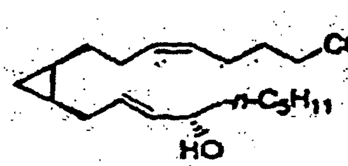 ; 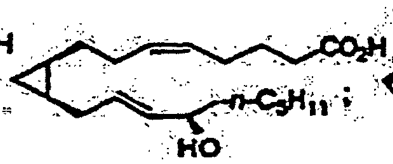 ; 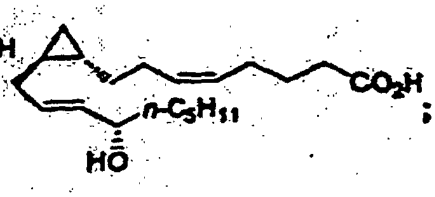 ;

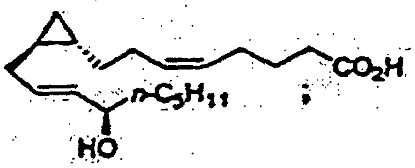 ; 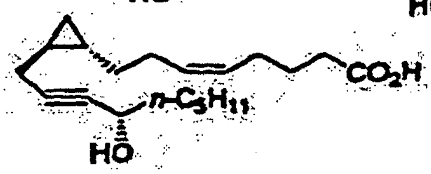 und

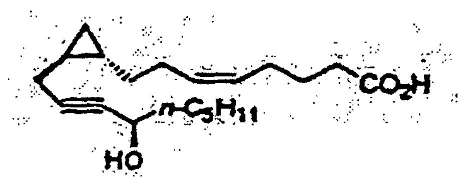

**15.** Verwendung nach Anspruch 9, wobei für die Verbindung der Formel I

$R^1$ $CO_2R$ ist, wobei R H oder ein ophthalmisch annehmbares kationisches Salz ist, oder $CO_2R$ einen ophthalmisch annehmbaren Esteranteil bildet;
A $L_5$-$A_2$-$L_3$ ist;
$L_3$ *trans*-$CH_2CH$=CH, *trans*-CH=$CHCH_2$, $CH_2C{\equiv}C$ oder $CH_2CH_2CH_2$ ist;
B *cis*-CH=CH oder C≡C ist;
D *trans*-CH=CH oder C≡C ist und
Y

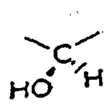 oder 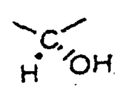

ist.

**16.** Verwendung nach Anspruch 15, wobei die Verbindung ausgewählt ist aus der Gruppe bestehend aus

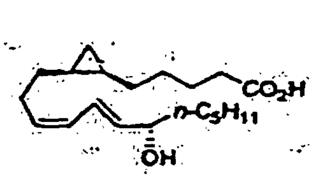 ; 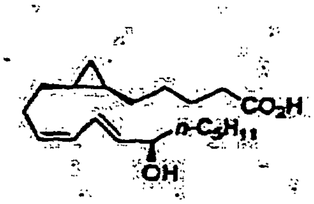 ; 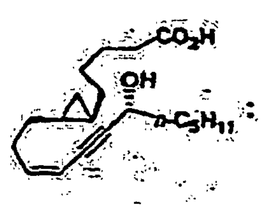 ;

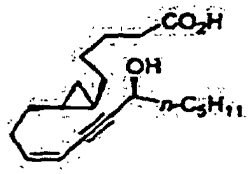 ; 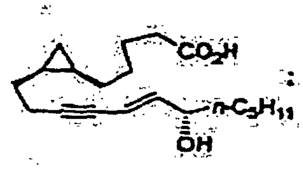 ; 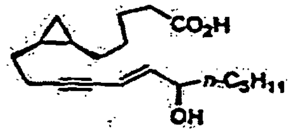 ;

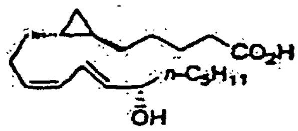 ; 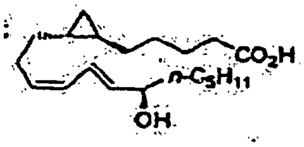 ; 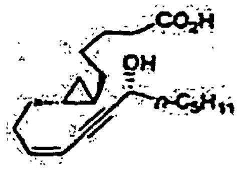 ;

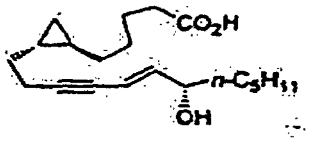 und 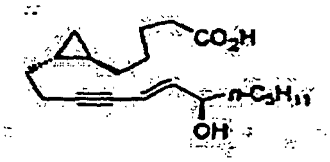

**17.** Verwendung nach Anspruch 9, wobei das trockene Auge und die anderen Leiden, die das Benetzen des Auges erfordern, Symptome von trockenem Auge sind, die mit einer refraktiven Chirurgie verbunden sind.

**18.** Verbindung der Formel I

$$n\text{-}C_5H_{11}\text{-}Y\text{-}A\text{-}CH_2\text{-}R^1 \qquad \mathbf{I}$$

I

worin

$R^1$ $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$ oder 2,3,4,5-Tetrazol-1-yl ist, wobei

R H oder ein pharmazeutisch annehmbares Kation ist oder $CO_2R$ einen pharmazeutisch annehmbaren Esteranteil bildet;
$NR^2R^3$, $NR^5R^6$ gleich oder verschieden sind und eine freie oder funktionell modifizierte Aminogruppe beinhalten;
$OR^4$ eine freie oder funktionell modifizierte Hydroxygruppe beinhaltet;
Hal F, Cl, Br oder I ist;
$R^{20}$ H, Alkyl, Acyl ist;
$R^{21}$ H oder ein pharmazeutisch annehmbares Kation ist oder $COSR^{21}$ einen pharmazeutisch annehmbaren Thioesteranteil bildet;

A $L_1$-$A_2$-$L_2$, $L_3$-$A_2$-$L_4$ oder $L_5$-$A_2$-$L_3$ ist,
$A_2$

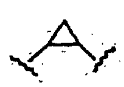

ist;
$L_1$ $CH_2$-B-D ist;

B und D gleich oder verschieden sind und $CH_2CH_2$, CH=CH oder C≡C bedeuten;
$L_2$ $CH_2$-K-$CH_2CH_2$ ist;
K $CH_2CH_2$, CH=CH oder C≡C ist;
$L_3$ $CH_2CH_2CH_2$, $CH_2CH$=CH, $CH_2C$≡C, CH=$CHCH_2$, C≡$CCH_2$ oder CH=C=CH ist;
$L_4$ X-$CH_2CH_2$ ist;
X $CH_2CH_2CH$=CH, $CH_2CH_2C$≡C, $CH_2CH_2CH_2CH_2$, $CH_2CH$=$CHCH_2$, $CH_2C$≡$CCH_2$, CH=$CHCH_2CH_2$, C≡$CCH_2CH_2$, $CH_2CH$=C=CH oder CH=C=$CHCH_2$ ist;
$L_5$ $CH_2CH_2$-B-D ist und
Y C(O) (d.h. eine Carbonylgruppe) ist oder Y

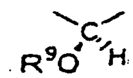
oder
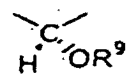

ist, wobei $R^9O$ eine freie oder funktionell modifizierte Hydroxygruppe bildet.

**19.** Verbindung nach Anspruch 18, wobei

$R^1$ $CO_2R$ ist, wobei R H oder ein ophthalmisch annehmbares kationisches Salz ist oder $CO_2R$ einen ophthalmisch annehmbaren Esteranteil bildet;
A $L_1$-$A_2$-$L_2$ ist;
B C≡C oder *cis*-CH=CH ist und D C≡C oder *trans*-CH=CH ist;
K *cis*-CH=CH ist und
Y

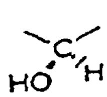
oder
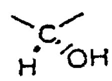

ist.

**20.** Verbindung nach Anspruch 19, wobei die Verbindung ausgewählt ist aus der Gruppe bestehend aus

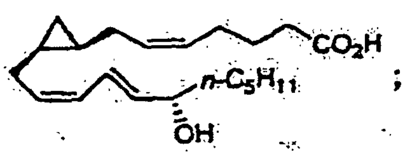
;
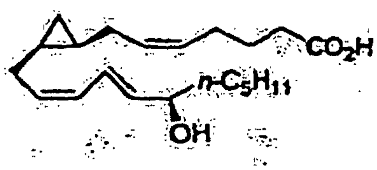
;
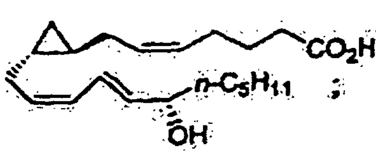
;
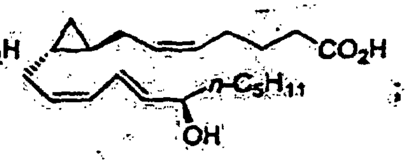
;
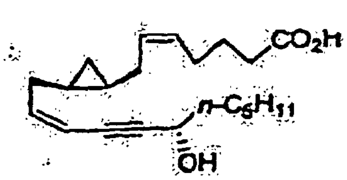
;
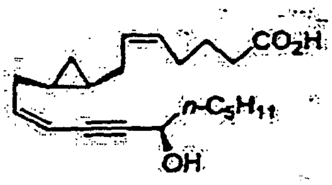
;
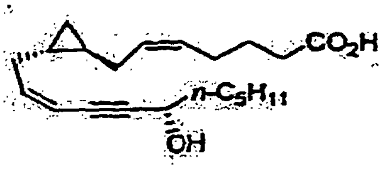
;

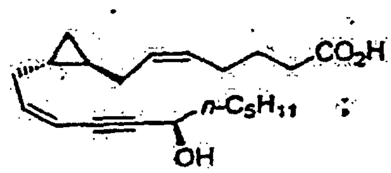 ; 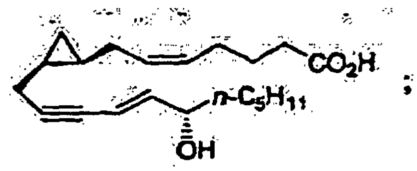 ;

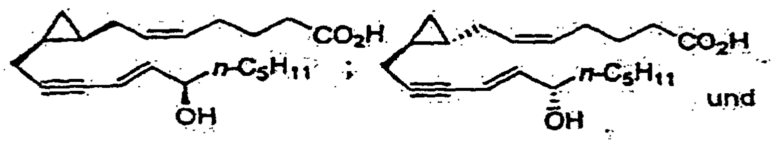 und 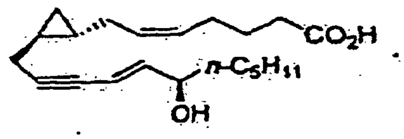

21. Verbindung nach Anspruch 18, wobei

R[1] $CO_2R$ ist, wobei R H oder ein ophthalmisch annehmbares kationisches Salz ist, oder $CO_2R$ einen ophthalmisch annehmbaren Esteranteil bildet;
A $L_3$-$A_2$-$L_4$ ist;
$L_3$ *trans*-$CH_2CH$=CH, *trans*-CH=$CHCH_2$ oder $CH_2C \equiv C$ ist;
X *trans*-$CH_2CH_2CH$=CH, $CH_2CH_2C \equiv C$, *trans*-$CH_2CH$=$CHCH_2$ oder *trans*-CH=$CHCH_2CH_2$ ist und
Y

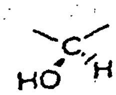 oder 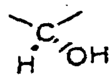

ist.

22. Verbindung nach Anspruch 21, wobei die Verbindung ausgewählt ist aus der Gruppe bestehend aus

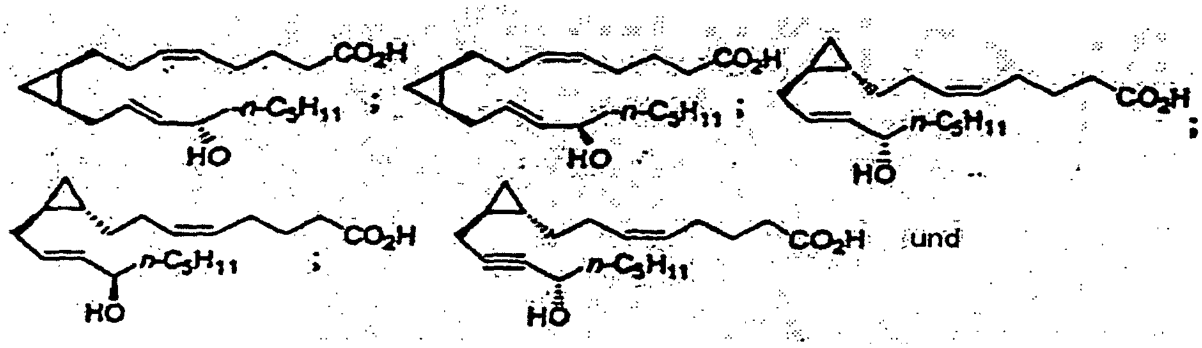

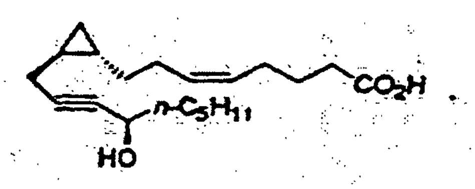

23. Verbindung nach Anspruch 18, wobei

R[1] $CO_2R$ ist, wobei R H oder ein ophthalmisch annehmbares kationisches Salz ist, oder $CO_2R$ einen ophthal-

misch annehmbaren Esteranteil bildet;
A $L_5$-$A_2$-$L_3$ ist;
$L_3$ *trans*-$CH_2CH{=}CH$, *trans*-$CH{=}CHCH_2$, $CH_2C{\equiv}C$ oder $CH_2CH_2CH_2$ ist;
B *cis*-$CH{=}CH$ oder $C{\equiv}C$ ist;
D *trans*-$CH{=}CH$ oder $C{=}C$ ist und
Y

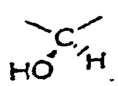
oder
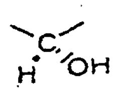

ist.

**24.** Verbindung nach Anspruch 23, wobei die Verbindung ausgewählt ist aus der Gruppe bestehend aus

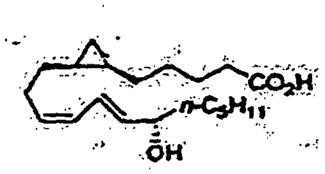
;
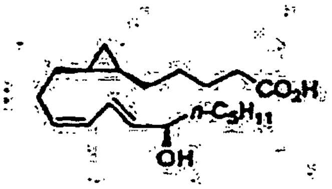
;
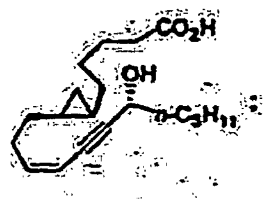
;
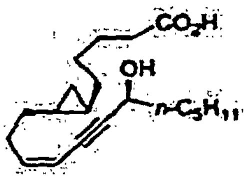
;
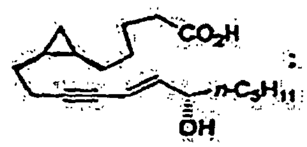
;
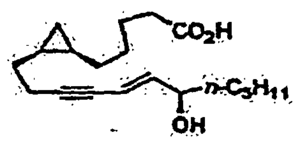
;
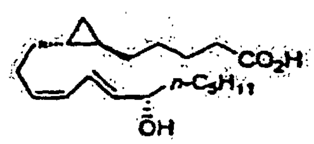
;
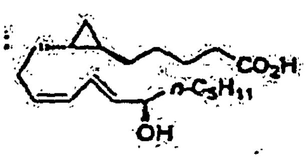
;
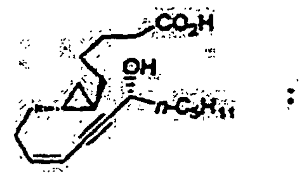
;
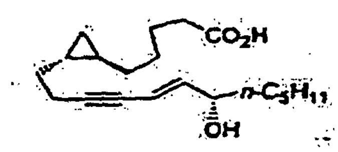
und
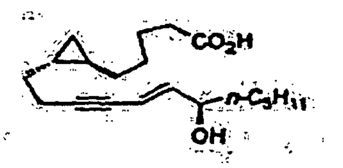
;

**Revendications**

**1.** Composition pour le traitement de l'oeil sec et d'autres affections nécessitant le mouillage de l'oeil comprenant un support pharmaceutiquement acceptable et une quantité pharmaceutiquement efficace d'un ou plusieurs composés de la formule I suivante :

$$n\text{-}C_5H_{11}\text{-}Y\text{-}A\text{-}CH_2\text{-}R^1 \qquad (I)$$

dans laquelle :

$R^1$ est un groupe $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$ ou 2,3,4,5-tétrazol-1-yle, où :

R est H ou un cation pharmaceutiquement acceptable, ou bien $CO_2R$ forme un fragment d'ester pharmaceutiquement acceptable;
$NR^2R^3$, $NR^5R^6$ sont identiques ou différents et comprennent un groupe amino libre ou modifié fonctionnellement;
$OR^4$ comprend un groupe hydroxy libre ou modifié fonctionnellement;
Hal est F, Cl, Br ou I;
$R^{20}$ est H, alkyle, acyle;
$R^{21}$ est H ou un cation pharmaceutiquement acceptable, ou bien $COSR^{21}$ forme un fragment de thioester pharmaceutiquement acceptable;

A est $L_1$-$A_2$-$L_2$, $L_3$-$A_2$-$L_4$ ou $L_5$-$A_2$-$L_3$;
$A_2$ est

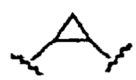

$L_1$ est $CH_2$-B-D;
B et D sont identiques ou différents et représentent un groupe $CH_2CH_2$, CH=CH ou C=C;
$L_2$ est $CH_2$-K-$CH_2CH_2$;
K est $CH_2CH_2$, CH=CH ou $C\equiv C$;
$L_3$ est $CH_2CH_2CH_2$, $CH_2CH$=CH, $CH_2C\equiv C$, CH=$CHCH_2$, $C\equiv CCH_2$ ou CH=C=CH;
$L_4$ est X-$CH_2CH_2$;
X est $CH_2CH_2CH$=CH, $CH_2CH_2C\equiv C$, $CH_2CH_2CH_2CH_2$, $CH_2CH$=$CHCH_2$, $CH_2C\equiv CCH_2$, CH=$CHCH_2CH_2$, $C\equiv CCH_2CH_2$, $CH_2CH$=C=CH ou CH=C=$CHCH_2$;
$L_5$ est $CH_2CH_2$-B-D; et
Y est un groupe C(O) (c'est-à-dire un groupe carbonyle) ou bien Y est un groupe

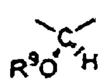

ou

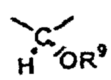

;

dans lequel $R^9O$ constitue un groupe hydroxy libre ou modifié fonctionnellement.

**2.** Composition suivant la revendication 1, dans laquelle pour le composé de formule I :

$R^1$ est un groupe $CO_2R$, où R est H ou un sel cationique ophtalmiquement acceptable, ou bien $CO_2R$ forme un fragment d'ester ophtalmiquement acceptable;
A est $L_1$-$A_2$-$L_2$;
B est un groupe $C\equiv C$ ou *cis*-CH=CH et D est un groupe $C\equiv C$ ou *trans*-CH=CH;
K est un groupe *cis*-CH=CH; et
Y est un groupe

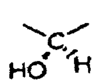

ou

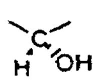

**3.** Composition suivant la revendication 2, dans laquelle le composé est choisi dans le groupe comprenant :

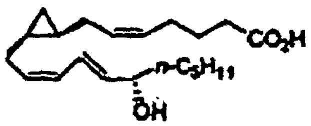 ; 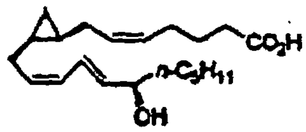 ; 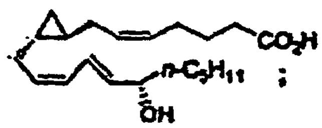 ;

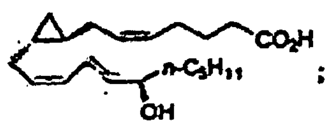 ; 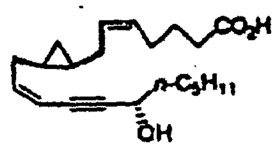 ; 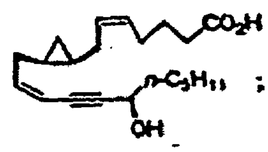 ;

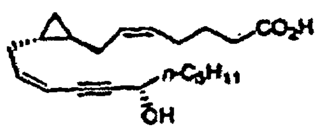 ; 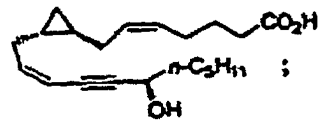 ; 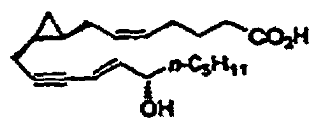

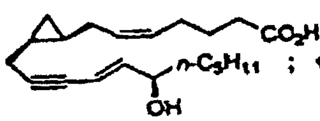 ; 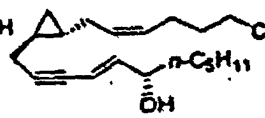 ; and 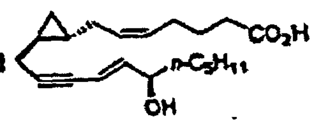 .

and = et.

**4.** Composition suivant la revendication 1, dans laquelle pour le composé de formule I :

$R^1$ est un groupe $CO_2R$, où R est H ou un sel cationique ophtalmiquement acceptable, ou bien $CO_2R$ forme un fragment d'ester ophtalmiquement acceptable;
A est $L_3$-$A_2$-$L_4$;
$L_3$ est un groupe *trans*-$CH_2CH=CH$, *trans*-$CH=CHCH_2$ ou $CH_2C\equiv C$;
X est un groupe *trans*-$CH_2CH_2CH=CH$, $CH_2CH_2C\equiv C$, *trans*-$CH_2CH=CHCH_2$ ou *trans*-$CH=CHCH_2CH_2$; et
Y est un groupe

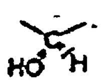 ou 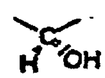

**5.** Composition suivant la revendication 1, dans laquelle le composé de formule I est choisi dans le groupe comprenant :

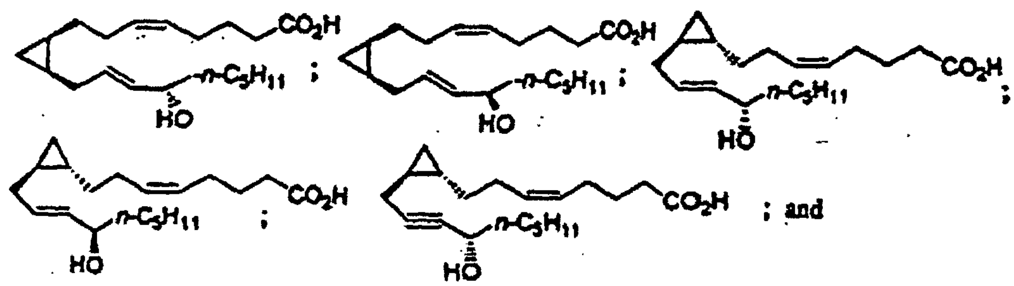

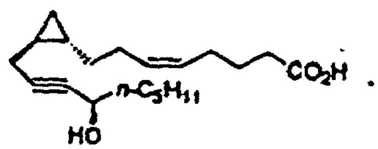

and = et.

**6.** Composition suivant la revendication 1, dans laquelle pour le composé de formule 1 :

$R^1$ est un groupe $CO_2R$, où R est H ou un sel cationique ophtalmiquement acceptable, ou bien $CO_2R$ forme un fragment d'ester ophtalmiquement acceptable;
A est $L_5$-$A_2$-$L_3$;
$L_3$ est un groupe *trans*-$CH_2CH$=CH, *trans*-CH=$CHCH_2$, $CH_2C{\equiv}C$, ou $CH_2CH_2CH_2$;
B est un groupe *cis*-CH=CH ou C≡C;
D est un groupe *trans*-CH=CH ou C≡C; et
Y est un groupe

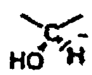
ou
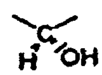

**7.** Composition suivant la revendication 6, dans laquelle le composé est choisi dans le groupe comprenant :

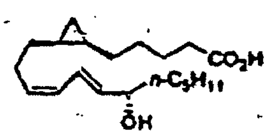 ; 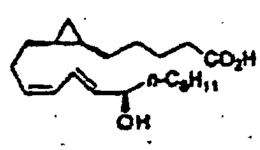 ; 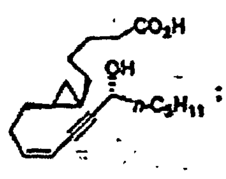 ;

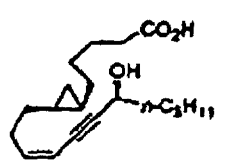 ; 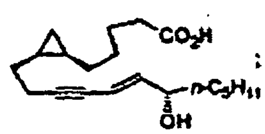 ; 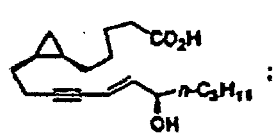 ;

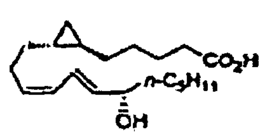 ; 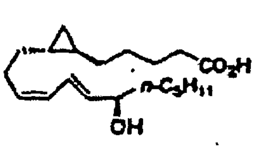 ; 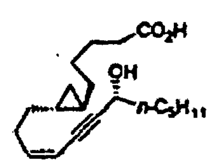

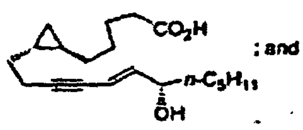
; and
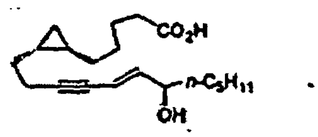
.

and = et.

**8.** Composition suivant la revendication 1, dans laquelle la composition est utilisable pour une administration topique à l'oeil.

**9.** Utilisation d'un ou plusieurs composés suivant la formule 1 :

$$n\text{-}C_5H_{11}\text{-}Y\text{-}A\text{-}CH_2\text{-}R^1 \qquad (I)$$

dans laquelle :

$R^1$ est un groupe $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$ ou 2,3,4,5-tétrazol-1-yle, où :

R est H ou un cation pharmaceutiquement acceptable, ou bien $CO_2R$ forme un fragment d'ester pharmaceutiquement acceptable;
$NR^2R^3$, $NR^5R^6$ sont identiques ou différents et comprennent un groupe amino libre ou modifié fonctionnellement;
$OR^4$ comprend un groupe hydroxy libre ou modifié fonctionnellement;
Hal est F, Cl, Br ou I;
$R^{20}$ est H, alkyle, acyle;
$R^{21}$ est H ou un cation pharmaceutiquement acceptable, ou bien $COSR^{21}$ forme un fragment de thioester pharmaceutiquement acceptable;

A est $L_1$-$A_2$-$L_2$, $L_3$-$A_2$-$L_4$ ou $L_5$-$A_2$-$L_3$;
$A_2$ est

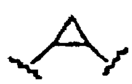

$L_1$ est $CH_2$-B-D;
B et D sont identiques ou différents et représentent un groupe $CH_2CH_2$, CH=CH ou C≡C;
$L_2$ est $CH_2$-K-$CH_2CH_2$;
K est $CH_2CH_2$, CH=CH ou C≡C;
$L_3$ est $CH_2CH_2CH_2$, $CH_2CH{=}CH$, $CH_2C{\equiv}C$, $CH{=}CHCH_2$, $C{\equiv}CCH_2$ ou CH=C=CH;
$L_4$ est X-$CH_2CH_2$;
X est $CH_2CH_2CH{=}CH$, $CH_2CH_2C{\equiv}C$, $CH_2CH_2CH_2CH_2$, $CH_2CH{=}CHCH_2$, $CH_2C{\equiv}CCH_2$, $CH{=}CHCH_2CH_2$, $C{\equiv}CCH_2CH_2$, $CH_2CH{=}C{=}CH$ ou $CH{=}C{=}CHCH_2$;
$L_5$ est $CH_2CH_2$-B-D; et
Y est un groupe C(O) (c'est-à-dire un groupe carbonyle) ou bien Y est un groupe

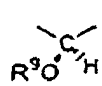
ou
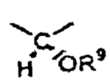
;

dans lequel $R^9O$ constitue un groupe hydroxy libre ou modifié fonctionnellement, pour la préparation d'une

composition pharmaceutique pour le traitement de l'oeil sec et d'autres affections nécessitant le mouillage de l'oeil chez les mammifères.

**10.** Utilisation suivant la revendication 9, dans laquelle le mammifère est un être humain et le composé est administré topiquement.

**11.** Utilisation suivant la revendication 9, dans laquelle pour le composé de formule I :

$R^1$ est un groupe $CO_2R$, où R est H ou un sel cationique ophtalmiquement acceptable, ou bien $CO_2R$ forme un fragment d'ester ophtalmiquement acceptable;
A est $L_1$-$A_2$-$L_2$;
B est un groupe C≡C ou *cis*-CH=CH et D est un groupe C≡C ou *trans*-CH=CH;
K est un groupe *cis*-CH=CH; et
Y est un groupe

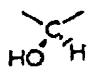

ou

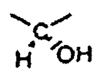

**12.** Utilisation suivant la revendication 1, dans laquelle le composé est choisi dans le groupe comprenant :

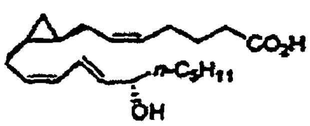 ; 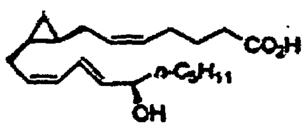 ; 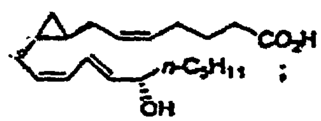 ;

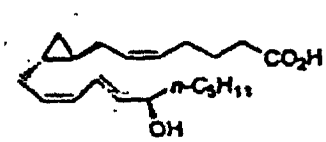 ; 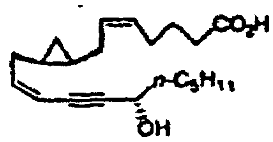 ; 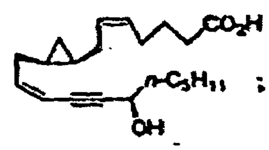 ;

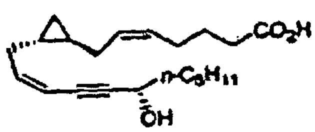 ; 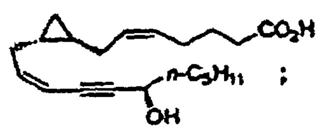 ; 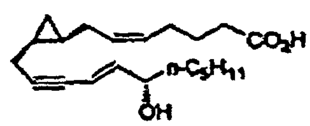

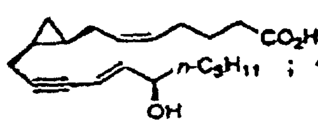 ; 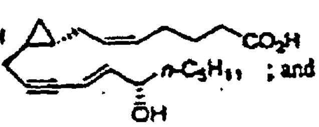 ; and 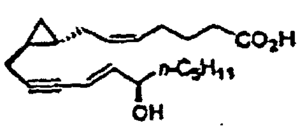 .

and = et.

**13.** Utilisation suivant la revendication 9, dans laquelle pour le composé de formule I :

$R^1$ est un groupe $CO_2R$, où R est H ou un sel cationique ophtalmiquement acceptable, ou bien $CO_2R$ forme un fragment d'ester ophtalmiquement acceptable;
A est $L_3$-$A_2$-$L_4$;

$L_3$ est un groupe *trans*-$CH_2CH=CH$, *trans*-$CH=CHCH_2$ ou $CH_2C\equiv C$;
X est un groupe *trans*-$CH_2CH_2CH=CH$, $CH_2CH_2C\equiv C$, *trans*-$CH_2CH=CHCH_2$ ou *trans*-$CH=CHCH_2CH_2$; et
Y est un groupe

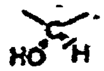 ou 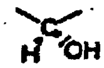

**14.** Utilisation suivant la revendication 9, dans laquelle le composé est choisi dans le groupe comprenant :

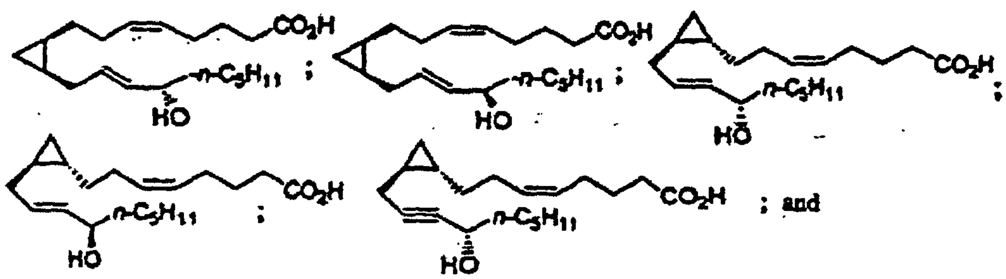

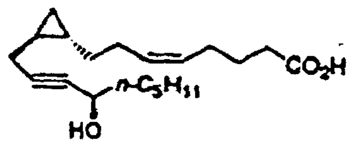

and = et.

**15.** Utilisation suivant la revendication 9, dans laquelle pour le composé de formule 1 :

$R^1$ est un groupe $CO_2R$, où R est H ou un sel cationique ophtalmiquement acceptable, ou bien $CO_2R$ forme un fragment d'ester ophtalmiquement acceptable;
A est $L_5$-$A_2$-$L_3$;
$L_3$ est un groupe *trans*-$CH_2CH=CH$, *trans*-$CH=CHCH_2$, $CH_2C\equiv C$, ou $CH_2CH_2CH_2$;
B est un groupe *cis*-$CH=CH$ ou $C\equiv C$;
D est un groupe *trans*-$CH=CH$ ou $C\equiv C$; et
Y est un groupe

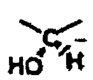 ou 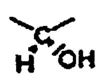

**16.** Utilisation suivant la revendication 15, dans laquelle le composé est choisi dans le groupe comprenant :

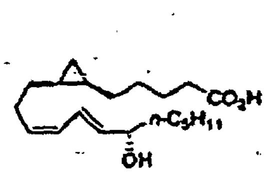 ; 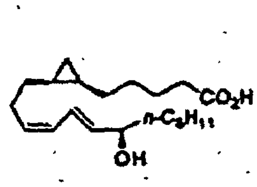 ; 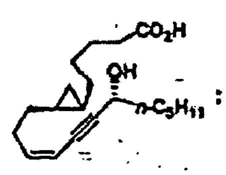 ;

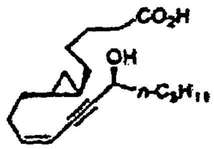 ; 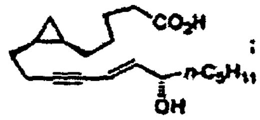 ; 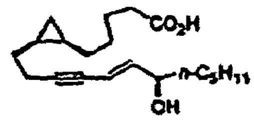 ;

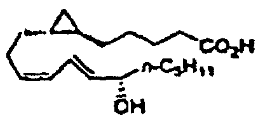 ; 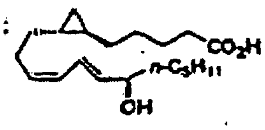 ; 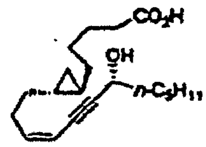 ;

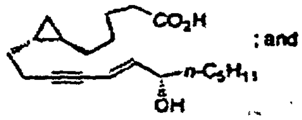 ; and 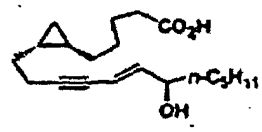 .

and = et.

**17.** Utilisation suivant la revendication 9, dans laquelle l'oeil sec et les autres affections nécessitant le mouillage de l'oeil sont des symptômes de l'oeil sec associés à une chirurgie réfringente.

**18.** Composé de formule I :

$$n\text{-}C_5H_{11}\text{-}Y\text{-}A\text{-}CH_2\text{-}R^1 \qquad (I)$$

dans laquelle :

$R^1$ est un groupe $CO_2R$, $CONR^2R^3$, $CH_2OR^4$, $CH_2NR^5R^6$, $CH_2N_3$, $CH_2Hal$, $CH_2NO_2$, $CH_2SR^{20}$, $COSR^{21}$ ou 2,3,4,5-tétrazol-1-yle, où:

R est H ou un cation pharmaceutiquement acceptable, ou bien $CO_2R$ forme un fragment d'ester pharmaceutiquement acceptable;
$NR^2R^3$, $NR^5R^6$ sont identiques ou différents et comprennent un groupe amino libre ou modifié fonctionnellement;
$OR^4$ comprend un groupe hydroxy libre ou modifié fonctionnellement;
Hal est F, Cl, Br ou I;
$R^{20}$ est H, alkyle, acyle;
$R^{21}$ est H ou un cation pharmaceutiquement acceptable, ou bien $COSR^{21}$ forme un fragment de thioester pharmaceutiquement acceptable; A est $L_1$-$A_2$-$L_2$, $L_3$-$A_2$-$L_4$ ou $L_5$-$A_2$-$L_3$;

$A_2$ est

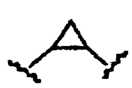

$L_1$ est $CH_2$-B-D;
B et D sont identiques ou différents et représentent un groupe $CH_2CH_2$, CH=CH ou C≡C;
$L_2$ est $CH_2$-K-$CH_2CH_2$;
K est $CH_2CH_2$, CH=CH ou C≡C;

$L_3$ est $CH_2CH_2CH_2$, $CH_2CH{=}CH$, $CH_2C{\equiv}C$, $CH{=}CHCH_2$, $C{\equiv}CCH_2$ ou $CH{=}C{=}CH$;
$L_4$ est X-$CH_2CH_2$;
X est $CH_2CH_2CH{=}CH$, $CH_2CH_2C{\equiv}C$, $CH_2CH_2CH_2CH_2$, $CH_2CH{=}CHCH_2$, $CH_2C{\equiv}CCH_2$, $CH{=}CHCH_2CH_2$, $C{\equiv}CCH_2CH_2$, $CH_2CH{=}C{=}CH$ ou $CH{=}C{=}CHCH_2$;
$L_5$ est $CH_2CH_2$-B-D; et
Y est un groupe C(O) (c'est-à-dire un groupe carbonyle) ou bien Y est un groupe

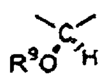 ou 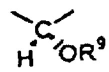 ;

dans lequel $R^9O$ constitue un groupe hydroxy libre ou modifié fonctionnellement.

**19.** Composé suivant la revendication 18, dans lequel :

$R^1$ est un groupe $CO_2R$, où R est H ou un sel cationique ophtalmiquement acceptable, ou bien $CO_2R$ forme un fragment d'ester ophtalmiquement acceptable;
A est $L_1$-$A_2$-$L_2$;
B est un groupe $C{\equiv}C$ ou cis-CH=CH et D est un groupe $C{\equiv}C$ ou *trans*-CH=CH:
K est un groupe *cis*-CH=CH; et

Y est un groupe

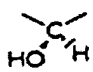 ou 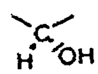

**20.** Composé suivant la revendication 19, dans lequel le composé est choisi dans le groupe comprenant :

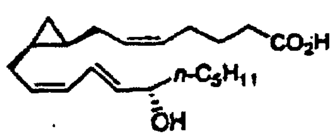 ; 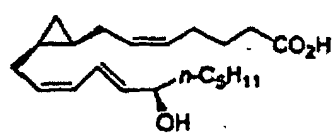 ;

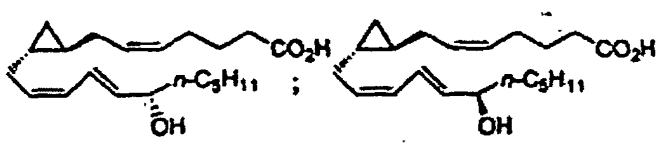

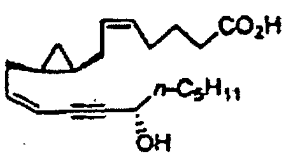 ; 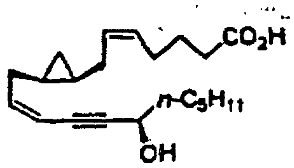 ; 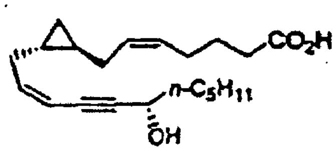 ;

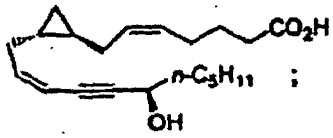 ; 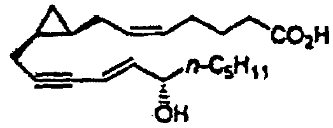 ;

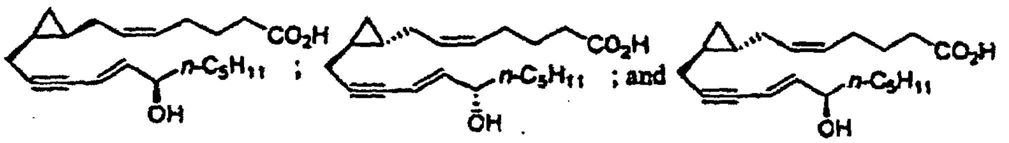

; and

and = et.

**21.** Composé suivant la revendication 18, dans lequel :

$R^1$ est un groupe $CO_2R$, où R est H ou un sel cationique ophtalmiquement acceptable, ou bien $CO_2R$ forme un fragment d'ester ophtalmiquement acceptable;
A est $L_3$-$A_2$-$L_4$;
$L_3$ est un groupe *trans*-$CH_2CH$=CH, *trans*-CH=$CHCH_2$ ou $CH_2C{\equiv}C$;
X est un groupe *trans*-$CH_2CH_2CH$=CH, $CH_2CH_2C{\equiv}C$, *trans*-$CH_2CH$=$CHCH_2$ ou *trans*-CH=$CHCH_2CH_2$; et
Y est un groupe

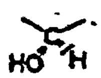

ou

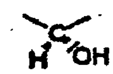

**22.** Composé suivant la revendication 21, dans lequel le composé est choisi dans le groupe comprenant :

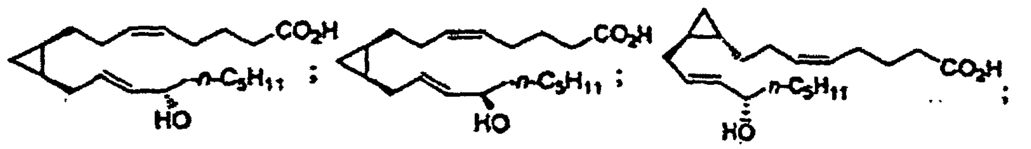

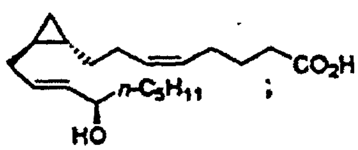

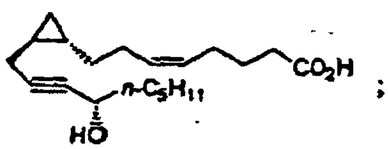

and

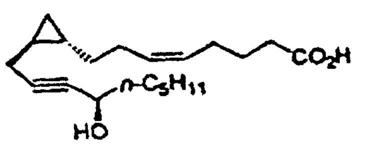

and = et.

**23.** Composé suivant la revendication 18, dans lequel :

$R^1$ est un groupe $CO_2R$, où R est H ou un sel cationique ophtalmiquement acceptable, ou bien $CO_2R$ forme un fragment d'ester ophtalmiquement acceptable;
A est $L_5$-$A_2$-$L_3$;
$L_3$ est un groupe *trans*-$CH_2CH$=CH, *trans*-CH=$CHCH_2$, $CH_2C{\equiv}C$, ou $CH_2CH_2CH_2$;
B est un groupe *cis*-CH=CH ou $C{\equiv}C$;
D est un groupe *trans*-CH=CH ou C=C; et

Y est un groupe

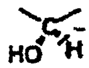 ou 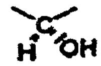

**24.** Composé suivant la revendication 23, dans lequel le composé est choisi dans le groupe comprenant :

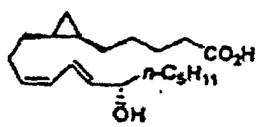 ; 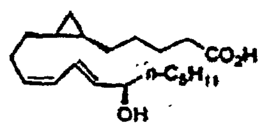 ; 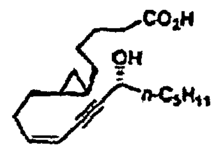 ;

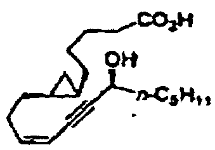 ; 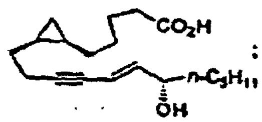 ; 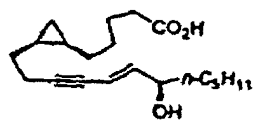 ;

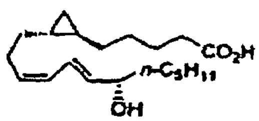 ; 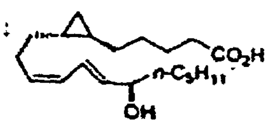 ; 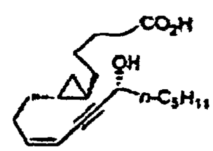 ;

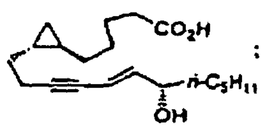 ; and 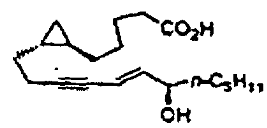

and = et.